# EUROPEAN PATENT APPLICATION

(11) **EP 3 342 840 A2**
(43) Date of publication of application: **04.07.2018**
(21) Application number: 17196370.5
(22) Date of filing: 16.12.2010
(51) Int. Cl.: C09K 3/30, C07C 21/18, C08J 9/04, C11D 3/24, C10M 131/04

(54) **COMPOSITIONS AND USES OF CIS-1,1,1,4,4,4-HEXAFLUORO-2-BUTENE**

(30) Priority: 16.12.2009 US 287033 P; 15.12.2010 US 968506
(62) Divisional of application: 10842581.0
(71) Applicant: Honeywell International Inc., Morris Plains, NJ 07950 (US)
(72) Inventor: Hulse, Ryan, Getzville, New York 14068 (US); Zyhowski, Gary John, Lancaster New York 14086 (US); Hofman, Bjiorn, Morris Plains, New Jersey 07950 (US); Williams, Dave, Morristown, New Jersey 07962-2245 (US); Knopeck, Gary, Morristown, New Jersey 07962 (US); Richard, Robert G., Morristown, New Jersey 07962 (US); Basu, Rajat, Morristown, New Jersey 07962 (US); Singh, Rajiv Ratna, Getzville, New York 14068 (US)
(74) Representative: Crooks, Elizabeth Caroline

(57) **Abstract**

This invention relates to compositions, methods and systems having utility in numerous applications, and in particular, uses for compositions containing the compound cis-1,1,1,4,4,4-hexafluoro-2-butene (Z-HFO-1336mzzm), which has the following structure:

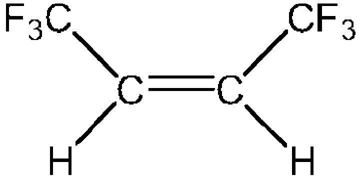

## Description

### CROSS-REFERENCE TO RELATED APPLICATION

This application claims benefit of U.S. Provisional Patent Application Serial No. 61/287,033 filed December 16, 2009, the disclosure of which is hereby incorporated herein by reference.

### FIELD OF THE INVENTION

This invention relates to compositions, methods and systems having utility in numerous applications, and in particular, uses for compositions containing the compound cis-1,1,1,4,4,4-hexafluoro-2-butene (Z-HFO-1336mzzm), which has the following structure:

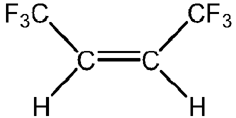

### BACKGROUND OF THE INVENTION

The compositions of the present invention are part of a continued search for the next generation of low global warming potential materials. Such materials must have low environmental impact, as measured by ultra-low global warming potential and zero ozone depletion potential.

### SUMMARY OF THE INVENTION

This invention relates to compositions, methods and systems having utility in numerous applications, and in particular, uses for compositions containing the compound cis-1,1,1,4,4,4-hexafluoro-2-butene (Z-HFO-1336mzzm), which has the following structure:

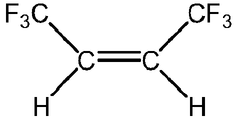

Embodiments of the present invention comprise the compound Z-HFO-1336mzzm, either alone or in combination with one or more other compounds as described in detail herein below. Preferably, mixtures containing the compound Z-HFO-1336mzzm are non-azeotropic.

It should be noted that it would be common and expected for a product designated as Z-HFO-1336mzzm to include a minor percentage, for example about 0.5 wt % up to about 5 wt % of other components, including particularly E-HFO-1336mzzm. When used herein, the term "consisting essentially of Z-HFO-1336mzzm" is intended to generally include such compositions. The terms "consist of" and "consisting of" as used herein, do not include such other components. All of the embodiments of the invention described herein may, if desired, be obtained in a substantially purified form, such that these embodiments preferably consist of only the actual components designated, other than minor (e.g., ppm) impurities.

The compositions of the present invention may be used in a wide variety of applications such as blowing agents, refrigerants, heating agents, power cycle agents, cleaning agents, aerosol propellants, sterilization agents, lubricants, flavor and fragrance extractants, flammability reducing agents, and flame suppression agents, to name a few preferred uses.

### DETAILED DESCRIPTION OF PREFERRED EMBODIMENTS

The compositions of the present invention all include the compound Z-HFO-1336mzzm. Certain embodiments of the invention, particularly those employed as blowing agent compositions or foamable compositions, can optionally include other ingredients, some of which are described in detail below.

In addition to the compound Z-HFO-1336mzzm, certain embodiments of the present invention are directed to compositions comprising, or consisting essentially of, at least one additional fluoroalkene containing from 2 to 6, preferably 3 to 5 carbon atoms, more preferably 3 to 4 carbon atoms, and in certain embodiments most preferably three carbon atoms, and at least one carbon-carbon double bond. The fluoroalkene compounds of the present invention are sometimes referred to herein for the purpose of convenience as hydrofluoro-olefins or "HFOs" if they contain at least one hydrogen.

Applicants have developed several compositions which include as an essential component the compound Z-HFO-1336mzzm and at least one additional compound such as HFOs, HFCs, HFEs, hydrocarbons, ethers, aldehydes, ketones, and others such as methyl formate, formic acid, trans-1,2 dichloroethylene, carbon dioxide, cis-HFO-1234ze + HFO-1225yez; mixtures of these plus water; mixtures of these plus CO2; mixtures of these trans 1,2-dichloroethylene (DCE); mixtures of these plus methyl formate; mixtures with cis-HFO-1234ze + CO2; mixtures with cis-HFO-1234ze + HFO-1225yez + CO2; and mixtures with cis-HFO-1234ze + HFC-245fa. In such compositions, the amount of the compound Z-HFO-1336mzzm may vary widely, including in all cases constituting the balance of the composition after all other components in composition are accounted for.

In certain preferred embodiments, the amount of the compound Z-HFO-1336mzzm in the composition can be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %.

The preferred compositions of the present invention are environmentally acceptable and do not to contribute to the depletion of the earth's stratospheric ozone layer. The compounds and compositions of the present invention have no substantial ozone depletion potential (ODP), preferably an ODP of not greater than about 0.5 and even more preferably an ODP of not greater than about 0.25, most preferably an ODP of not greater than about 0.1; and/or a global warming potential (GWP) of not greater than about 150, and even more preferably, a GWP of not greater than about 50.

As used herein, ODP is defined in the "Scientific Assessment of Ozone Depletion, 2002," a report of the World Meteorological association, incorporated here by reference.
As used herein, GWP is defined relative to that of carbon dioxide and over a 100 year time horizon, and defined in the same reference as for the ODP mentioned above.

Preferred compositions of this type are described below in Table 1 (with all percentages being in percent by weight and being understood to be proceeded by the word "about").

**Table 1 - Blend Compositions**

| Compound mixed with Z-HFO-1336mzzm | | Preferred Ranges wt % | More Preferred Ranges wt % | Most Preferred Ranges wt % |
|---|---|---|---|---|
| HFOs | | | | |
| | cis-HFO-1234ze | 1 to 99 | 1 to 70 | 1 to 50 |
| | HFO-1234yf | 1 to 99 | 1 to 70 | 1 to 50 |
| | HFO 1225yeZ | 1 to 99 | 1 to 70 | 1 to 50 |
| | HFO 1225yeE | 1 to 99 | 1 to 70 | 1 to 50 |
| | HFO1225yc | 1 to 99 | 1 to 70 | 1 to 50 |
| | HFO-1233zd | 1 to 99 | 20 to 80 | 30 to 70 |
| | HFC-1233xf | 1 to 99 | 20 to 80 | 30 to 70 |
| | CF3CH=CHCF3 (E) | 1 to 99 | 1 to 70 | 1 to 50 |
| | (CF3)2CFCH=CHF (E&Z) | 1 to 99 | 1 to 70 | 1 to 50 |
| | (CF3)2CFCH=CF2 | 1 to 99 | 1 to 70 | 1 to 50 |
| | CF3CHFC=CHF (E & Z) | 1 to 99 | 1 to 70 | 1 to 50 |
| | (C2F5)(CF3)C=CH2 | 1 to 99 | 1 to 70 | 1 to 50 |

| HFCs | | | | |
|---|---|---|---|---|
| | HFC-245fa | 1 to 99 | 1 to 70 | 1 to 25 |
| | HFC-245eb | 1 to 99 | 1 to 70 | 1 to 25 |
| | HFC-245ca | 1 to 99 | 1 to 70 | 1 to 30 |
| | HFC-227ea | 1 to 99 | 1 to 70 | 1 to 10 |
| | HFC-236ea | 1 to 99 | 1 to 70 | 1 to 20 |
| | HFC-236fa | 1 to 99 | 1 to 70 | 1 to 5 |
| | HFC-134a | 1 to 99 | 1 to 70 | 1 to 15 |
| | HFC-134 | 1 to 99 | 1 to 70 | 1 to 20 |
| | HFC-152a | 1 to 99 | 1 to 70 | 1 to 20 |
| | HFC-32 | 1 to 99 | 1 to 70 | 1 to 25 |
| | HFC-125 | 1 to 99 | 1 to 70 | 1 to 10 |
| | HFC-143a | 1 to 99 | 1 to 70 | 1 to 10 |
| | HFC-365mfc | 1 to 99 | 1 to 70 | 1 to 25 |
| | HFC-161 | 1 to 99 | 1 to 70 | 1 to 20 |
| | HFC-43-10mee | 1 to 99 | 1 to 70 | 1 to 15 |

| HFEs | | | | |
|---|---|---|---|---|
| | CHF2-O-CHF2 | 1 to 99 | 1 to 70 | 1 to 50 |
| | CHF2-O-CH2F | 1 to 99 | 1 to 70 | 1 to 50 |
| | CH2F-O-CH2F | 1 to 99 | 1 to 70 | 1 to 50 |
| | CH2F-O-CH3 | 1 to 99 | 1 to 70 | 1 to 50 |
| | CYCLO-CF2-CH2-CF2-O | 1 to 99 | 1 to 70 | 1 to 50 |
| | CYCLO-CF2-CF2-CH2-O | 1 to 99 | 1 to 70 | 1 to 50 |
| | CHF2-O-CF2-CHF2 | 1 to 99 | 1 to 70 | 1 to 50 |
| | CF3-CF2-O-CH2F | 1 to 99 | 1 to 70 | 1 to 50 |
| | CHF2-O-CHF-CF3 | 1 to 99 | 1 to 70 | 1 to 50 |

| Compound mixed with Z-HFO-1336mzzm | | Preferred Ranges wt % | More Preferred Ranges wt % | Most Preferred Ranges wt % |
|---|---|---|---|---|
| | CHF2-O-CF2-CHF2 | 1 to 99 | 1 to 70 | 1 to 50 |
| | CH2F-O-CF2-CHF2 | 1 to 99 | 1 to 70 | 1 to 50 |
| | CF3-O-CF2-CH3 | 1 to 99 | 1 to 70 | 1 to 50 |
| | CHF2-CHF-O-CHF2 | 1 to 99 | 1 to 70 | 1 to 50 |
| | CF3-O-CHF-CH2F | 1 to 99 | 1 to 70 | 1 to 50 |
| | CF3-CHF-O-CH2F | 1 to 99 | 1 to 70 | 1 to 50 |
| | CF3-O-CH2-CHF2 | 1 to 99 | 1 to 70 | 1 to 50 |
| | CHF2-O-CH2-CF3 | 1 to 99 | 1 to 70 | 1 to 50 |
| | CH2F-CF2-O-CH2F | 1 to 99 | 1 to 70 | 1 to 50 |
| | CHF2-O-CF2-CH3 | 1 to 99 | 1 to 70 | 1 to 50 |
| | CHF2-CF2-O-CH3 (254pc) | 1 to 99 | 1 to 70 | 1 to 50 |
| | CH2F-O-CHF-CH2F | 1 to 99 | 1 to 70 | 1 to 50 |
| | CHF2-CHF-O-CH2F | 1 to 99 | 1 to 70 | 1 to 50 |
| | CF3-O-CHF-CH3 | 1 to 99 | 1 to 70 | 1 to 50 |
| | CF3-CHF-O-CH3 | 1 to 99 | 1 to 70 | 1 to 50 |
| | CHF2-O-CH2-CHF2 | 1 to 99 | 1 to 70 | 1 to 50 |
| | CF3-O-CH2-CH2F | 1 to 99 | 1 to 70 | 1 to 50 |
| | CF3-CH2-O-CH2F | 1 to 99 | 1 to 70 | 1 to 50 |
| | CF2H-CF2-CF2-O-CH3 | 1 to 99 | 1 to 70 | 1 to 50 |

| Hydrocarbons | | | | |
|---|---|---|---|---|
| | Propane | 1 to 99 | 20 to 95 | 40 to 95 |
| | Butane | 1 to 99 | 20 to 95 | 40 to 95 |
| | Isobutane | 1 to 99 | 20 to 95 | 40 to 95 |
| | n-pentane (high HFO) | 1 to 99 | 50 to 99 | 60 to 99 |
| | n-pentane (high n-pentane) | 1 to 99 | 1 to 30 | 1 to 20 |
| | Isopentane (High HFO) | 1 to 99 | 50 to 99 | 60 to 99 |
| | Isopentane (High isopentane) | 1 to 99 | 1 to 30 | 1 to 20 |
| | Neopentane (High HFO) | 1 to 99 | 50 to 99 | 60 to 99 |
| | Neopentane (High neopentane) | 1 to 99 | 1 to 30 | 1 to 20 |
| | Cyclopentane (High HFO) | 1 to 99 | 50 to 99 | 60 to 99 |
| | Cyclopentane (High cyclopentane) | 1 to 99 | 1 to 30 | 1 to 20 |
| | n-hexane | 1 to 99 | 20 to 95 | 40 to 95 |
| | Isohexane | 1 to 99 | 20 to 95 | 40 to 95 |
| | Heptane | 1 to 99 | 20 to 95 | 40 to 95 |

| Ethers | | | | |
|---|---|---|---|---|
| | Dimethylether | 1 to 99 | 10 to 90 | 10 to 80 |
| | Methylethylether | 1 to 99 | 10 to 90 | 10 to 80 |

| Compound mixed with Z-HFO-1336mzzm | | Preferred Ranges wt % | More Preferred Ranges wt % | Most Preferred Ranges wt % |
|---|---|---|---|---|
| | diethyl ether | 1 to 99 | 10 to 90 | 10 to 80 |
| | Methylpropylether | 1 to 99 | 10 to 90 | 10 to 80 |
| | Methylisopropylether | 1 to 99 | 10 to 90 | 10 to 80 |
| | Ethylpropylether | 1 to 99 | 10 to 90 | 10 to 80 |
| | Ethylisopropylether | 1 to 99 | 10 to 90 | 10 to 80 |
| | Dipropylether | 1 to 99 | 10 to 90 | 10 to 80 |
| | Diisopropylether | 1 to 99 | 10 to 90 | 10 to 80 |
| | Dimethyloxymethane | 1 to 99 | 10 to 90 | 10 to 80 |
| | Diethoxymethane | 1 to 99 | 10 to 90 | 10 to 80 |
| | Dipropoxymethane | 1 to 99 | 10 to 90 | 10 to 80 |
| | Dibutoxymethane | 1 to 99 | 10 to 90 | 10 to 80 |

| Aldehydes | | | | |
|---|---|---|---|---|
| | Formaldehyde | 1 to 99 | 10 to 90 | 10 to 80 |
| | Acetaldehyde | 1 to 99 | 10 to 90 | 10 to 80 |
| | Propanal | 1 to 99 | 10 to 90 | 10 to 80 |
| | Butanal | 1 to 99 | 10 to 90 | 10 to 80 |
| | Isobutanal | 1 to 99 | 10 to 90 | 10 to 80 |

| Ketones | | | | |
|---|---|---|---|---|
| | Acetone | 1 to 99 | 10 to 90 | 10 to 80 |
| | Methylethylketone | 1 to 99 | 10 to 90 | 10 to 80 |
| | Methylisobutylketone | 1 to 99 | 10 to 90 | 10 to 80 |
| | | | | |

| Others | | | | |
|---|---|---|---|---|
| | methyl formate | 1 to 99 | 10 to 90 | 10 to 80 |
| | formic acid | 1 to 99 | 10 to 90 | 10 to 80 |
| | Trans-1,2 dichloroethylene | 1 to 99 | 1 to 50 | 1 to 30 |
| | Carbon dioxide | 1 to 99 | 10 to 90 | 10 to 80 |
| | cis-HFO-1234ze + HFO-1225yez | 1 to 25 / to 50 | 1 to 20 / 1 to 25 | 1 to 15 / 1 to 10 |
| | Mixtures of any of the above plus water | 1 to 50% H2O | 1 to 25% H2O | 1 to 15 % H2O |
| | Mixtures of any of the above plus CO2 | 1 to 50% CO2 | 1 to 25% CO2 | 1 to 15% CO2 |
| | Mixtures of any of the above plus trans 1,2-dichloroethylene (DCE) | 1 to 50% DCE | 1 to 25% DCE | 1 to 15% DCE |
| | Mixtures of any of the above plus methyl formate | 1 to 50% MF | 1 to 25% MF | 1 to 15% MF |

| Compound mixed with Z-HFO-1336mzzm | | Preferred Ranges wt % | More Preferred Ranges wt % | Most Preferred Ranges wt % |
|---|---|---|---|---|
| | (MF) | | | |
| | Mixtures with cis-HFO-1234ze + CO2 | 1 to 25 / to 50 | 1 to 20 / 1 to 25 | 1 to 15 / 1 to 10 |
| | Mixtures with cis-HFO-1234ze + HFO-1225yez + CO2 | 1 to 25 / to 50 | 1 to 20 / 1 to 25 | 1 to 15 / 1 to 10 |
| | Mixtures with cis-HFO-1234ze + HFC-245fa | 1 to 25 / to 50 | 1 to 20 / 1 to 25 | 1 to 15 / 1 to 10 |

### USES OF THE COMPOSITIONS

As described above, the compositions of the present invention may be used in a wide variety of applications as substitutes for CFCs and for compositions containing less desirable HCFCs. For example, the present compositions are useful as blowing agents, refrigerants, heating agents, power cycle agents, cleaning agents, aerosol propellants, sterilization agents, lubricants, flavor and fragrance extractants, flammability reducing agents, and flame suppression agents, to name a few preferred uses. Each of these uses will be discussed in greater detail below.

### BLOWING AGENTS

Thus, the present invention includes methods and systems which include using Z-HFO-1336mzzm as a blowing agent, optionally with one or more optional additional compounds which include, but are not limited to, other compounds which also act as blowing agents (hereinafter referred to for convenience but not by way of limitation as co-blowing agents), surfactants, polyols, catalysts, flame retardants, polymer modifiers, colorants, dyes, solubility enhancers, rheology modifiers, plasticizing agents, fillers, nucleating agents, viscosity reduction agents, vapor pressure modifiers, stabilizers, and the like. Preferred blends for blowing agents used for foams, especially spray foams and panel foams include blends of Z-HFO-1336mzzm with hydrocarbons (especially the pentanes, including cyclopentane), and with each of 245fa, 365mfc and 1233zd. While the cis isomer of HFO-1336mzzm is preferred, it is anticipated that the trans isomer and/or mixtures of the isomers, including the racemate, will be useful in certain foam types.

For this use, the amount of the compound Z-HFO-1336mzzm in the composition of the invention can be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use of the composition of the invention.

In certain preferred embodiments, dispersing agents, cell stabilizers, surfactants and other additives may also be incorporated into the blowing agent compositions of the present invention. Certain surfactants are optionally but preferably added to serve as cell stabilizers. Some representative materials are sold under the names of DC-193, B-8404, and L-5340 which are, generally, polysiloxane polyoxyalkylene block co-polymers such as those disclosed in U.S. Pat. Nos. 2,834,748, 2,917,480, and 2,846,458, each of which is incorporated herein by reference. Other optional additives for the blowing agent mixture may include flame retardants such as tri(2-chloroethyl)phosphate, tri(2-chloropropyl)phosphate, tri(2,3-dibromopropyl)-phosphate, tri(1,3-dichloro-propyl)phosphate, diammonium phosphate, various halogenated aromatic compounds, antimony oxide, aluminum trihydrate, polyvinyl chloride, and the like. With respect to nucleating agents, all known compounds and materials having nucleating functionality are available for use in the present invention, including particularly talc.

Of course other compounds and/or components that modulate a particular property of the compositions (such as cost for example) may also be included in the present compositions, and the presence of all such compounds and components is within the broad scope of the invention.

The co-blowing agent in accordance with the present invention can comprise a physical blowing agent, a chemical blowing agent (which preferably in certain embodiments comprises water) or a blowing agent having a combination of physical and chemical blowing agent properties.

Although it is contemplated that a wide range of co-blowing agents may be used in accordance with the present invention, in certain embodiments it is preferred that the blowing agent compositions of the present invention include one or more HFCs as co-blowing agents, more preferably one or more C1-C4 HFCs, and/or one or more hydrocarbons, more preferably C4-C6 hydrocarbons. For example, with respect to HFCs, the present blowing agent compositions may include one or more of difluoromethane (HFC-32), fluoroethane (HFC-161), difluoroethane (HFC-152), trifluoroethane (HFC-143), tetrafluoroethane (HFC-134), pentafluoroethane (HFC-125), pentafluoropropane (HFC-245), hexafluoropropane (HFC-236), heptafluoropropane (HFC-227ea), pentafluorobutane (HFC-365mfc), hexafluorobutane (HFC-356) and all isomers of all such HFC's.

With respect to hydrocarbons, the present blowing agent compositions may include in certain preferred embodiments, for example, iso, normal and/or cyclopentane for thermoset foams and butane or isobutane for thermoplastic foams. Of course other materials, such as water, CO2, CFCs (such as trichlorofluoromethane (CFC-11) and dichlorodifluoromethane (CFC-12)), hydrochlorocarbons (HCCs such as dichloro-ethylene (preferably trans-1,2-dichloroethylene), ethyl chloride and chloropropane), HCFCs, C1-C5 alcohols (such as, for example, ethanol and/or propanol and/or butanol), C1-C4 aldehydes, C1-C4 ketones, C1-C4 ethers (including ethers (such as dimethyl ether and diethyl ether), diethers (such as dimethoxy methane and diethoxy methane)), and methyl formate including combinations of any of these may be included, although such components are contemplated to be not preferred in many embodiments due to negative environmental impact.

In certain embodiments, one or more of the following HFC isomers arc preferred for use as co-blowing agents in the compositions of the present invention:
1,1,1,2,2-pentafluoroethane (HFC-125)
1,1,2,2-tetrafluoroethane (HFC-134)
1,1,1,2-tetrafluoroethane (HFC-134a)
1,1-difluoroethane (HFC-152a)
1,1,1,2,3,3,3-heptafluoropropane (HFC-227ea)
1,1,1,3,3,3-hexafluoropropane (HFC-236fa)
1,1,1,3,3-pentafluoropropane (HFC-245fa) and
1,1,1,3,3-pentafluorobutane (HFC-365mfc).

The relative amount of any of the above noted additional co-blowing agents, as well as any additional components which may be included in present compositions, can vary widely within the general broad scope of the present invention according to the particular application for the composition, and all such relative amounts are considered to be within the scope hereof.

In certain embodiments it is preferred that the blowing agent composition of the present invention comprise at least one co-blowing agent and an amount of Z-HFO-1336mzzm sufficient to produce a blowing agent composition which is overall nonflammable.

The blowing agent compositions of the present invention may include the compound Z-HFO-1336mzzm in widely ranging amounts. It is generally preferred, however, that for preferred compositions for use as blowing agents in accordance with the present invention, Z-HFO-1336mzzm is present in an amount that is at least about 1% by weight, more preferably at least about 5% by weight, and even more preferably at least about 15% by weight, of the composition.

In certain preferred embodiments, the blowing agent comprises at least about 50% by weight of the present blowing agent compound(s), and in certain embodiments the blowing agent consists essentially of Z-HFO-1336mzzm. In this regard it is noted that the use of one or more co-blowing agents is consistent with the novel and basic features of the present invention. For example, it is contemplated that water will be used as either a co-blowing or in combination with other co-blowing agents (such as, for example, pentane, particularly cyclopentane) in a large number of embodiments.

In certain preferred embodiments, the blowing agent composition comprises from about 30% to about 95% by weight of Z-HFO-1336mzzm and from about 5% to about 90% by weight, more preferably from about 5% to about 65% by weight of co-blowing agent. In certain of such embodiments the co-blowing agent comprises, and preferably consists essentially of, H2O, HFCs, hydrocarbons, alcohols (preferably C2, C3 and/or C4 alcohols), CO2, and combinations of these.

In preferred embodiments in which the co-blowing agent comprises H2O, the composition comprises H2O in an amount of from about 5% by weight to about 50% by weight of the total blowing agent composition, more preferably from about 10% by weight to about 40% by weight, and even more preferably of from about 10% to about 20% by weight of the total blowing agent.

In preferred embodiments in which the co-blowing agent comprises CO2, the composition comprises CO2 in an amount of from about 5% by weight to about 60% by weight of the total blowing agent composition, more preferably from about 20% by weight to about 50% by weight, and even more preferably of from about 40% to about 50% by weight of the total blowing agent.

In preferred embodiments in which the co-blowing agent comprises alcohols, (preferably C2, C3 and/or C4 alcohols), the composition comprises alcohol in an amount of from about 5% by weight to about 40% by weight of the total blowing agent composition, more preferably from about 10% by weight to about 40% by weight, and even more preferably of from about 15% to about 25% by weight of the total blowing agent.

For compositions which include HFC co-blowing agents, the HFC co-blowing agent (preferably C2, C3, C4 and/or C5 HFC), and even more preferably difluoromethane (HFC-152a) (HFC-152a being particularly preferred for extruded thermoplastics) and/or pentafluoropropane (HFC-245)), is preferably present in the composition in amounts of from of from about 5% by weight to about 80% by weight of the total blowing agent composition, more preferably from about 10% by weight to about 75% by weight, and even more preferably of from about 25% to about 75% by weight of the total blowing agent. Furthermore, in such embodiments, the HFC is preferably C2-C4 HFC, and even more preferably C3 HFC, with penta-fluorinated C3 HFC, such as HFC-245fa, being highly preferred in certain embodiments.

For compositions which include HC co-blowing agents, the HC co-blowing agent (preferably C3, C4 and/or C5 HC) is preferably present in the composition in amounts of from of from about 5% by weight to about 80% by weight of the total blowing agent composition, and even more preferably from about 20% by weight to about 60% by weight of the total blowing agent.

### BLOWING AGENT EXAMPLE

This example demonstrates the performance of Z-HFO-1336mzzm used in combination with hydrocarbon co-blowing agents, and in particular the utility of compositions comprising, or consisting essentially of, Z-HFO-1336mzzm and cyclopentane co-blowing agents in rigid polyurethane insulation foams.

A generic refrigerator appliance-type polyurethane foam formulation (foam forming mixture) is provided. The polyol blend consisted of commercial polyol(s), catalyst(s), surfactant(s), and water. Standard commercial polyurethane processing equipment is used for the foam forming process. A blowing agent combination is formed comprising, or consisting essentially of, Z-HFO-1336mzzm in a concentration of approximately 50 mole percent, and cyclopentane in a concentration of approximately 50 mole percent of the total physical blowing agent. The physical blowing agents can be added individually to the polyol blend or can be pre-blended prior to introduction to the polyol blend.

This example illustrates the physical the thermal conductivity properties of all the resulting foams are suitable for commercial use of these blowing agent combinations.

### FOAMABLE COMPOSITIONS

One embodiment of the present invention provides foamable compositions. As is known to those skilled in the art, foamable compositions generally include one or more components capable of forming foam. As used herein, the term "foam foaming agent" is used to refer to a component, or a combination on components, which are capable of forming a foam structure, preferably a generally cellular foam structure. The foamable compositions of the present invention include such component(s) and a blowing agent compound, preferably Z-HFO-1336mzzm.

For this use, the amount of the compound Z-HFO-1336mzzm in the composition of the invention can be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use of the composition of the invention.

In certain embodiments, the one or more components capable of forming foam comprise a thermosetting composition capable of forming foam and/or foamable compositions. Examples of thermosetting compositions include polyurethane and polyisocyanurate foam compositions, and also phenolic foam compositions. This reaction and foaming process may be enhanced through the use of various additives such as catalysts and surfactant materials that serve to control and adjust cell size and to stabilize the foam structure during formation. Furthermore, is contemplated that any one or more of the additional components described above with respect to the blowing agent compositions of the present invention could be incorporated into the foamable composition of the present invention. In such thermosetting foam embodiments, one or more of the present compositions are included as or part of a blowing agent in a foamable composition, or as a part of a two or more part foamable composition, which preferably includes one or more of the components capable of reacting and/or foaming under the proper conditions to form a foam or cellular structure.

In certain other embodiments of the present invention, the one or more components capable of foaming comprise thermoplastic materials, particularly thermoplastic polymers and/or resins. Examples of thermoplastic foam components include polyolefins, such as for example monovinyl aromatic compounds of the formula Ar-CHCH2 wherein Ar is an aromatic hydrocarbon radical of the benzene series such as polystyrene (PS). Other examples of suitable polyolefin resins in accordance with the invention include the various ethylene resins including the ethylene homopolymers such as polyethylene and ethylene copolymers, polypropylene (PP) and polyethylene-terephthalate (PET). In certain embodiments, the thermoplastic foamable composition is an extrudable composition.

It is contemplated that all presently known and available methods and systems for forming foam are readily adaptable for use in connection with the present invention. For example, the methods of the present invention generally require incorporating a blowing agent in accordance with the present invention into a foamable or foam forming composition and then foaming the composition, preferably by a step or series of steps which include causing volumetric expansion of the blowing agent in accordance with the present invention.

In general, it is contemplated that the presently used systems and devices for incorporation of blowing agent and for foaming arc readily adaptable for use in accordance with the present invention. In fact, it is believed that one advantage of the present invention is the provision of an improved blowing agent which is generally compatible with existing foaming methods and systems.

Thus, it will be appreciated by those skilled in the art that the present invention comprises methods and systems for foaming all types of foams, including thermosetting foams, thermoplastic foams and formed-in-place foams. Thus, one aspect of the present invention is the use of the present blowing agents in connection conventional foaming equipment, such as polyurethane foaming equipment, at conventional processing conditions. The present methods therefore include polyol premix type operations, blending type operations, third stream blowing agent addition, and blowing agent addition at the foam head.

With respect to thermoplastic foams, the preferred methods generally comprise introducing a blowing agent in accordance with the present invention into a thermoplastic material, preferably thermoplastic polymer such as polyolefin, and then subjecting the thermoplastic material to conditions effective to cause foaming. For example, the step of introducing the blowing agent into the thermoplastic material may comprise introducing the blowing agent into a screw extruder containing the thermoplastic, and the step of causing foaming may comprise lowering the pressure on the thermoplastic material and thereby causing expansion of the blowing agent and contributing to the foaming of the material.

It will be appreciated by those skilled in the art, especially in view of the disclosure contained herein, that the order and manner in which the blowing agent of the present invention is formed and/or added to the foamable composition does not generally affect the operability of the present invention. For example, in the case of extrudable foams, it is possible that the various components of the blowing agent, and even the components of the foamable composition, be not be mixed in advance of introduction to the extrusion equipment, or even that the components are not added to the same location in the extrusion equipment. Moreover, the blowing agent can be introduced either directly or as part of a premix, which is then further added to other parts of the foamable composition.

Thus, in certain embodiments it may be desired to introduce one or more components of the blowing agent at first location in the extruder, which is upstream of the place of addition of one or more other components of the blowing agent, with the expectation that the components will come together in the extruder and/or operate more effectively in this manner. Nevertheless, in certain embodiments, two or more components of the blowing agent are combined in advance and introduced together into the foamable composition, either directly or as part of premix which is then further added to other parts of the foamable composition.

### FOAMS

One embodiment of the present invention relates to methods of forming foams, especially panel foams and spray foams, and preferably such foams made from polyurethane and polyisocyanurate. The methods generally comprise providing a blowing agent composition of the present inventions, adding (directly or indirectly) the blowing agent composition to a foamable composition, and reacting the foamable composition under the conditions effective to form a foam or cellular structure, as is well known in the art. Any of the methods well known in the art, such as those described in "Polyurethanes Chemistry and Technology," Volumes I and II, Saunders and Frisch, 1962, John Wiley and Sons, New York, N.Y., which is incorporated herein by reference, may be used or adapted for use in accordance with the foam embodiments of the present invention.

For this use, the amount of the compound Z-HFO-1336mzzm in the composition of the invention can be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use of the composition of the invention.

In general, such preferred methods comprise preparing polyurethane or polyisocyanurate foams by combining an isocyanate, a polyol or mixture of polyols, a blowing agent or mixture of blowing agents comprising one or more of the present compositions, and other materials such as catalysts, surfactants, and optionally, flame retardants, colorants, or other additives.

It is convenient in many applications to provide the components for polyurethane or polyisocyanurate foams in pre-blended formulations. Most typically, the foam formulation is pre-blended into two components. The isocyanate and optionally certain surfactants and blowing agents comprise the first component, commonly referred to as the "A" component. The polyol or polyol mixture, surfactant, catalysts, blowing agents, flame retardant, and other isocyanate reactive components comprise the second component, commonly referred to as the "B" component. Accordingly, polyurethane or polyisocyanurate foams are readily prepared by bringing together the A and B side components either by hand mix for small preparations and, preferably, machine mix techniques to form blocks, slabs, laminates, pour-in-place panels and other items, spray applied foams, froths, and the like. Optionally, other ingredients such as fire retardants, colorants, auxiliary blowing agents, and even other polyols can be added as one or more additional streams to the mix head or reaction site. Most preferably, however, they are all incorporated into one B-component as described above.

The present methods and systems also include forming a one component foam, preferably polyurethane foam, containing a blowing agent in accordance with the present invention. In certain preferably embodiments, a portion of the blowing agent is contained in the foam forming agent, preferably by being dissolved in a foam forming agent which is liquid at the pressure within the container, a second portion of the blowing agent is present as a separate gas phase. In such systems, the contained/dissolved blowing agent performs, in large part, to cause the expansion of the foam, and the separate gas phase operates to impart propulsive force to the foam forming agent.

Such one component systems are typically and preferably packaged in a container, such as an aerosol type can, and the blowing agent of the present invention thus preferably provides for expansion of the foam and/or the energy to transport the foam/foamable material from the package, and preferably both. In certain embodiments, such systems and methods comprise charging the package with a fully formulated system (preferably isocyanate/polyol system) and incorporating a gaseous blowing agent in accordance with the present invention into the package, preferably an aerosol type can.

It is contemplated also that in certain embodiments it may be desirable to utilize the present compositions when in the supercritical or near supercritical state as a blowing agent.

The present invention also relates to all foams, including but not limited to closed cell foam, open cell foam, spray foams, panel foams, rigid foam, flexible foam, integral skin and the like, prepared from a polymer foam formulation containing a blowing agent comprising, or consisting essentially of, Z-HFO-1336mzzm, either alone or in combination with one or more other compounds.

Applicants have found that one advantage of the foams, and particularly thermoset foams such as polyurethane foams, in accordance with the present invention is the ability to achieve, preferably in connection with thermoset foam embodiments, exceptional thermal performance, such as can be measured by the K-factor or lambda, particularly and preferably under low temperature conditions, as shown in Figure 1. Although it is contemplated that the present foams, particularly thermoset foams of the present invention, may be used in a wide variety of applications, in certain preferred embodiments the present invention comprises appliance foams in accordance with the present invention, including refrigerator foams, freezer foams, refrigerator/freezer foams, panel foams, and other cold or cryogenic manufacturing applications.

The foams in accordance with the present invention, in certain preferred embodiments, provide one or more exceptional features, characteristics and/or properties, including: thermal insulation efficiency (particularly for thermoset foams), dimensional stability, compressive strength, aging of thermal insulation properties, all in addition to the low ozone depletion potential and low global warming potential associated with many of the preferred blowing agents of the present invention. In certain highly preferred embodiments, the present invention provides thermoset foam, including such foam formed into foam articles, which exhibit improved thermal conductivity relative to foams made using the same blowing agent (or a commonly used blowing agent HFC-245fa) in the same amount but without the compound Z-HFO-1336mzzm.

In other preferred embodiments, the present foams exhibit improved mechanical properties relative to foams produced with blowing agents outside the scope of the present invention. For example, certain preferred embodiments of the present invention provide foams and foam articles having a compressive strength which is superior to, and preferably at least about 10 relative percent, and even more preferably at least about 15 relative percent greater than a foam produced under substantially identical conditions by utilizing a blowing agent consisting of cyclopentane.

Furthermore, it is preferred in certain embodiments that the foams produced in accordance with the present invention have compressive strengths that are on a commercial basis comparable to the compressive strength produced by making a foam under substantially the same conditions except wherein the blowing agent consists of HFC-245fa. In certain preferred embodiments, the foams of the present invention exhibit a compressive strength of at least about 12.5% yield (in the parallel and perpendicular directions), and even more preferably at least about 13% yield in each of said directions.

### PANEL FOAM EXAMPLES

Panel foams made using a 80/20 wt% Z-HFO-1336mzzm/cyclopentane blend as the blowing agent provides significantly better physical property and thermal insulation value than foams made with either Z-HFO-1336mzzm/iso-pentane blend or Z-HFO-1336mzzm/n-pentane blends as the blowing agent. In addition, panel foams made with a blowing agent blend of 80/20 wt % Z-HFO-1336mzzm/cyclopentane had considerably shorter tack free time. With nearly identical foam density, foam made with Z-HFO-1336mzzm/cyclopentane blend had the highest compressive strength. Foams made with the Z-HFO-1336mzzm/cyclopentane blend also demonstrated lower thermal conductivity and better retention of thermal insulation value after aging than foam with either Z-HFO-1336mzzm/isopentane blend or Z-HFO-1336mzzm/n-pentane blend.

Panel foams made with 80/20 wt% of Z-HFO-1336mzzm and hydrocarbons as the blowing agent were prepared as follows. The polyol master batch composition is shown in Table 2 while the generic panel foam formulations with corresponding amounts of blowing agents are listed in Table 3.

**Table 2 - Polyol Master Batch Composition**

| Component | Weight (%) | php. |
|---|---|---|
| Voranol 490 Polyol | 51.38 | 65.00 |
| Terate 4020 Polyol | 27.67 | 35.00 |
| Polycat 8 Catalyst | 1.58 | 2.00 |
| DABCO DC-193 Surfactant | 1.19 | 1.50 |
| TMCP Flame Retardant | 17.39 | 22.00 |
| Water | 0.79 | 1.00 |
| Total | 100.00 | 126.50 |

**Table 3 - Generic Panel Foam Formulations with Z-HFO-1336mzzm/Hydrocarbon**

| | Cyclopentane | Isopentane | N-pentane |
|---|---|---|---|
| Mol.% of 1336mzzm | 63.0 | 63.7 | 63.7 |
| Mol.% of Hydrocarbon | 37.0 | 36.3 | 36.3 |
| Wt.% of 1336mzzm | 80.0 | 80.0 | 80.0 |
| Wt.% of Hydrocarbon | 20.0 | 20.0 | 20.0 |

| Polyol Blend | | | |
|---|---|---|---|
| Master Batch | 126.5 | 126.5 | 126.5 |
| 1336mzzm | 20.9 | 21.1 | 21.1 |
| Hydrocarbon | 5.2 | 5.3 | 5.3 |
| Total | 152.6 | 152.9 | 152.9 |

| Isocyanate | | | |
|---|---|---|---|
| Lupranate M20 | 127.3 | 127.3 | 127.3 |
| NCO Index | 110 | 110 | 110 |

**Table 4 - Physical Properties of Z-HFO-1336mzzm/Hydrocarbon Foams**

| | Cyclopentane | Isopentane | N-pentane |
|---|---|---|---|
| Reactivity, second | | | |
| Cream Time | 23 | 22 | 23 |
| Gel Time | 70 | 75 | 70 |
| Tack-Free Time | 103 | 140 | 140 |
| Density, lb/ft3 | | | |
| Foam Density | 1.88 | 1.89 | 1.91 |

| Compressive Strength, psi | | | |
|---|---|---|---|
| Perpendicular | 24.4 | 22.6 | 23.2 |
| Parallel | 12.2 | 11.7 | 11.2 |
| Ratio, Perpendicular/Parallel | 2.0 | 1.9 | 2.1 |
| Ratio, Perpendicular/Density | 13.0 | 12.0 | 12.1 |
| Ratio, Parallel/Density | 6.5 | 6.2 | 5.9 |

| Dimensional Stability, vol.% changed | | | |
|---|---|---|---|
| 01 Day | | | |
| Cold, -29°C | -0.23 | -0.25 | -0.31 |
| Hot/Humid, | | | |
| 70°C/95%R.H. | 15.12 | 10.43 | 12.26 |

| 07 Day | | | |
|---|---|---|---|
| Cold, -29°C | -0.33 | -0.22 | -0.19 |
| Hot/Humid, | | | |
| 70°C/95%R.H. | 26.87 | 20.35 | 15.70 |

### Reactivity

Similar cream time and gel time are recorded for all three foams. However, the tack-free time of foam with Z-HFO-1336mzzm/cyclopentane blend is significantly shorter than foams with Z-HFO-1336mzzm/isopentane blend or Z-HFO-1336mzzm/n-pentane blend as blowing agent. At the tack-free time, the outer surface of the foam loses its stickiness and the foam can be removed from the mold. Foam with Z-HFO- 1336mzzm/cyclopentane blend may provide faster demold time than those two foams with other hydrocarbon blends.

### Compressive Strength

The perpendicular-to-parallel compressive strength ratios of all three foams are similar; however, the perpendicular compressive strength to density ratio and the parallel compressive strength to density ratio of the foam with Z-HFO-1336mzzm/cyclopentane blend are significantly higher than the other two foams. The results indicated that, with identical foam density, foam which is blown by Z-HFO-1336mzzm/cyclopentane blend will provide the highest compressive strength among all three foams.

### Dimensional Stability

No significant difference was found in the dimensional stability evaluation at - 29°C after 7 days. The results for hot/humid dimensional stability evaluation are inconclusive at this stage.

### Thermal Conductivity

Initially, foam with Z-HFO-1336mzzm/cyclopentane blend demonstrates slightly better insulation value than the other two foams at all temperatures evaluated. After the foams were aged for 8 days, the difference in thermal conductivity appears to be more significant. Compared to the foams with Z-HFO-1336mzzm/isopentane or Z-HFO-1336mzzm/n-pentane blend, foam with Z-HFO-1336mzzm/cyclopentane blend provides better retention of insulation value after aging.

### SPRAY FOAM EXAMPLES

Spray prepared with Z-HFO-1336mzzm, 1233zd(E), 30/70 mole % blend of 1233zd(E) /1336mzzm and 70/30 mole % blend of 1233zd(E)/1336mzzm had equivalent density. The thermal conductivity data from these foams do not demonstrate the anticipated linear relationship. In fact, foam prepared with a 70/30 mole % 1233zd(E)/1336mzzm and 30/70 mole % 1233zd(E)/1336mzzm have improved k-factors and superior aging to those made with 1233zd(E). This is an unanticipated result.

Spray foams with Z-HFO-1336mzzm, 1233zd(E), 30/70 mole % blend of 1233zd(E)/1336mzzm and 70/30 mole % blend of 1233zd(E)/1336mzzm as blowing agent were prepared as follows. The polyol master batch composition is shown in Table 5 while the generic spray foam formulations with corresponding amounts of blowing agents are listed in Table 6. The foams were prepared with a 3 second pour time and 8 second mix time. The raw materials temperatures were 50 °F polyol/ 70°F MDI.

**Table 5 - Polyol Master Batch Composition**

| Component | Lot Numbers | Php |
|---|---|---|
| Jeffol R- 470 x | VC03019501 | 50.00 |
| Tcratc 4020 | MY4020-18 | 43.75 |
| Dicthylcnc glycol | B11+024 | 6.25 |
| DABCO DC-193 | 0001580875 | 1.25 |
| Dabco DMEA | 258009 | 2.00 |
| Antiblaze AB80 | 122 | 12.50 |
| Water | | 1.25 |
| Total | | 117.00 |

**Table 6 - Spray Foam Formulations**

| | 1233zd(E) | 1233zd(E)/ 1336mzzm 70/30 mole % | 1233zd(E)/ 1336mzzm 30/70 mole % | 1336mzzm |
|---|---|---|---|---|
| Mol.% of 1336mzzm | 0 | 30 | 70 | 100 |
| Mol.% of 1233zd(E) | 100 | 70 | 30 | 0 |
| Moles of 1336mzzm | 0 | 0.061 | 0.143 | 0.204 |
| Moles of 1233zd(E) | 0.204 | 0.143 | 0.061 | 0 |

| Polyol Blend | | | | |
|---|---|---|---|---|
| Master Batch | 117.0 | 117.0 | 117.0 | 117.0 |
| 1336mzzm | 0 | 10.0 | 23.5 | 33.5 |
| 1233zd(E) | 26.5 | 18.6 | 7.9 | 0 |
| Total | 143.5 | 145.6 | 148.4 | 150.5 |

| Isocyanate | | | | |
|---|---|---|---|---|
| Lupranate M20 | 137.38 | 137.38 | 137.38 | 137.38 |
| NCO Index | 110 | 110 | 110 | 110 |

### Reactivity

The relationship between cream time, gel time and tack free time are anticipated. They are equivalent for all foams prepared.

**Table 7 - Foam Reactivity**

| | 1233zd(E) | 1233zd(E)/ 1336mzzm 70/30 mole % | 1233zd(E)/ 1336mzzm 30/70 mole % | 1336mzzm |
|---|---|---|---|---|
| Reactivity, second | | | | |
| Cream Time | 15 | 15 | 12 | 12 |
| Gel Time | 35 | 34 | 33 | 38 |
| Tack-Free Time | 45 | 45 | 44 | 48 |

### Foam Quality/ Cell size/ Open Cell Content

The foams prepared were well mixed and equivalent in quality. The block density of the foams produced is similar as is the ratio of block to core density. Block density is density of the squared foam prior to sample cutting. Core density is density of the k- factor sample taken from the middle of the sample. This is anticipated since the foams were prepared with equivalent moles of blowing agents.

**Table 8 - Foam Quality: Density/ Cell Size/ Open Cell Content**

| | 1233zd(E) | 1233zd(E)/ 1336mzzm 70/30 mole % | 1233zd(E)/ 1336mzzm 30/70 mole % | 1336mzzm |
|---|---|---|---|---|
| Density, lb/ft³ | | | | |
| Foam Density- Block* | 1.8 | 1.78 | 1.81 | 1.79 |
| Foam Density- Core* | 1.83 | 1.71 | 1.77 | 1.82 |
| Ratio Block/ Core | | 1.04 | | |
| Density | 0.98 | | 1.02 | 0.98 |

| Open Cell Content, % Density, lb/ft³ | | | | |
|---|---|---|---|---|
| Average Cell Size, mm Average Cell Size | 0.2 | 0.2 | 0.2 | 0.2 |

### Compressive Strength

There is not a linear relationship between blowing agent concentration and perpendicular and parallel compressive strength. However, the variance form the linear relationship is considered minimal.

**Table 9 - Foam Compressive Strength**

| | 1233zd(E) | 1233zd(E)/ 1336mzzm 70/30 mole % | 1233zd(E)/ 1336mzzm 30/70 mole % | 1336mzzm |
|---|---|---|---|---|
| Compressive Strength, psi Perpendicular | 13.163 | 12.869 | 12.132 | 12.38 |
| Parallel | 25.678 | 22.870 | 23.392 | 26.95 |
| Ratio, Perpendicular/Parallel | 0.51 | 0.56 | 0.52 | 0.46 |

### Dimensional Stability

The addition of Z-HFO-1336mzzm to 1233zd(E) foam negatively impacts the dimensional stability of the foam in both cold and hot humid environments. It increases the shrinkage in the cold environment. In the hot humid environment the foams prepared from the blends swell more that the foams prepared from either of the neat compounds.

**Table 10 - Foam Dimensional Stability**

| | 1233zd(E) | 1233zd(E)/ 1336mzzm 70/30 mole % | 1233zd(E)/ 1336mzzm 30/70 mole % | 1336mzzm |
|---|---|---|---|---|
| Dimensional Stability, vol.% changed Cold, -29°C | | | | |
| 1 Day | 0.097 | -0.115 | 0.067 | -0.001 |
| 7 Day | -0.164 | -0.450 | -0.514 | 0.608 |
| 14 Day | -0.134 | -0.347 | -0.133 | -0.108 |

| Hot/Humid, 70°C/95%R.H. | | | | |
|---|---|---|---|---|
| 1 Day | 5.950 | 4.804 | 7.458 | 5.326 |
| 7 Day | 15.724 | 20.898 | 31.006 | 17.720 |
| 14 Day | 23.598 | 30.314 | 45.523 | 28.501 |

### Thermal Conductivity

The thermal conductivity of foams prepared with these blends are significantly improved over those made with 1233zd(E). Not only are they improved, the improvement is nonlinear in relationship to the amount of Z-HFO-1336mzzm added to the blowing agent blend. It is particularly interesting that the improvement at the low mean temperatures is significant and not 1233zd(E) concentration dependant. In addition, it is notable that the foam prepared from the blends age slower than the 1233zd(E) and the Z-HFO-1336mzzm foams.

**Table 11 - Foam Thermal Conductivity**

| | 1233zd(E) | 1233zd(E)/ 1336mzzm 70/30 mole % | 1233zd(E)/ 1336mzzm 30/70 mole % | 1336mzzm |
|---|---|---|---|---|
| Initial | | | | |
| 40 °F | 0.1357 | 0.1328 | 0.1320 | 0.1356 |
| 75 °F | 0.1540 | 0.1485 | 0.1459 | 0.1432 |
| 110 °F | 0.1744 | 0.1667 | 0.1643 | 0.1612 |

| 8 Day | | | | |
|---|---|---|---|---|
| 40 °F | 0.1415 | 0.1360 | 0.1367 | 0.1397 |
| 75 °F | 0.1595 | 0.1527 | 0.1506 | 0.1472 |
| 110 °F | 0.1798 | 0.1719 | 0.1711 | 0.1672 |

| 14 Day | | | | |
|---|---|---|---|---|
| 40 °F | 0.1431 | 0.1371 | 0.1380 | 0.1421 |
| 75 °F | 0.1626 | 0.1558 | 0.1505 | 0.1479 |
| 110 °F | 0.1838 | 0.1772 | 0.1697 | 0.1667 |

Foams prepared with Z-HFO-1336mzzm, 245fa, 30/70 mole % blend of 245fa / Z-HFO-1336mzzm and 70/30 mole % blend of 245fa/Z-HFO-1336mzzm had equivalent density. The dimensional stability and thermal conductivity data form these foams do not demonstrate the anticipated linear relationship. In fact, foam prepared with a 70/30 mole % 245fa/ Z-HFO-1336mzzm have improved k-factors and superior aging to those made with 245fa. This is an unexpected result.

Foams were prepared with Z-HFO-1336mzzm, 245fa, 30/70 mole % blend of 245fa/ Z-HFO-1336mzzm and 70/30 mole % blend of 245fa/ Z-HFO-1336mzzm as the blowing agents. The polyol master batch composition is shown above in Table 5 while the generic spray foam formulations with corresponding amounts of blowing agents are listed below in Table 12. The foams were prepared with and 3 second pour time and 8 second mix time. The raw materials temperatures were 50 °F polyol/ 70°F MDI.

**Table 12 - Polyol Master Batch Composition**

| Component | Lot Numbers | php |
|---|---|---|
| Jcffol R- 470 x | VC03019501 | 50.00 |
| Terate 4020 | MY4020-18 | 43.75 |
| Diethylene glycol | B11+024 | 6.25 |
| DABCO DC-193 | 0001580875 | 1.25 |
| Dabco DMEA | 258009 | 2.00 |
| Antiblaze AB80 | 122 | 12.50 |
| Water | | 1.25 |
| Total | | 117.00 |

**Table 13 - Generic Spray Foam Formulations**

| | 245fa | 245 fa/ 1336mzzm | 245 fa/ 1336mzzm | 1336mzzm |
|---|---|---|---|---|
| | | 70/30 mole % | 30/70 mole % | |
| Mol.% of 1336mzzm | 0 | 30 | 70 | 100 |
| Mol.% of 245fa | 100 | 70 | 30 | 0 |
| Moles of 1336mzzm | 0 | 0.061 | 0.143 | 0.204 |
| Moles of 245fa | 0.204 | 0.143 | 0.061 | 0 |
| | | | | |
| Polyol Blend | | | | |
| | | | | |
| Master Batch | 117.0 | 117.0 | 117.0 | 117.0 |
| 1336mzzm | 0 | 10.0 | 23.5 | 33.5 |
| 245fa | 27.3 | 19.2 | 8.2 | 0 |
| Total | 144.3 | 146.2 | 148.7 | 150.5 |
| | | | | |
| Isocyanate | | | | |
| | | | | |
| Lupranate M20 | 137.38 | 137.38 | 137.38 | 137.38 |
| NCO Index | 10 | 110 | 110 | 110 |

### Physical Properties

### Reactivity

The relationship between cream time, gel time and tack free time are anticipated. It would be anticipated that the addition of a high boiler such as Z-HFO-1336mzzm would extend the cream and gel time of the foam system.

**Table 14 - Foam Reactivity**

| | 245fa | 245fa/ 1336mzzm 70/30 mole % | 245fa/ 1336mzzm 30/70 mole % | 1336mzzm |
|---|---|---|---|---|
| Reactivity, second Cream Time | Immediate | 10 | 12 | 12 |
| Gel Time | 29 | 35 | 42 | 38 |
| Tack-Free Time | 43 | 48 | 52 | 48 |

### Foam Quality/ Cell size/ Open Cell Content

The foams prepared were well mixed and equivalent in quality. The block density of the foams produced is similar as is the ratio of block to core density. This is anticipated since the foams were prepared with equivalent moles of blowing agents.

**Table 15 - Foam Quality: Density/ Cell Size/ Open Cell Content**

| | 245fa | 245 fa/ 1336mzzm 70/30 mole % | 245fa/ 1336mzzm 30/70 mole % | 1336mzzm |
|---|---|---|---|---|
| Density, lb/ft3 | | | | |
| Foam Density- Block | 1.78 | 1.79 | 1.79 | 1.79 |
| Foam Density- Core | 1.75 | 1.94 | 1.86 | 1.82 |
| Ratio Block/ Core | | 0.92 | | |
| Density | 1.02 | | 0.96 | 0.98 |

| Open Cell Content, % Density, lb/ft3 | | | | |
|---|---|---|---|---|
| Average Cell Size, mm | | | | |
| Average Cell Size | 0.2 | 0.2 | 0.2 | 0.2 |
| Compressive Strength | | | | |

There is a near linear relationship in the perpendicular-to-parallel compressive strength ratios. The ratio decrease with reduction in the use of 245fa.

**Table 16 - Foam Compressive Strength**

| | 245fa | 245fa/ 1336mzzm 70/30 mole % | 245fa/ 1336mzzm 30/70 mole % | 1336mzzm |
|---|---|---|---|---|
| Compressive Strength, psi | | | | |
| Perpendicular | 13.24 | 12.32 | 12.48 | 12.38 |
| Parallel | 20.66 | 24.78 | 29.25 | 26.95 |
| Ratio, Perpendicular/Parallel | 0.64 | 0.50 | 0.43 | 0.46 |

### Dimensional Stability

The addition of Z-HFO-1336mzzm to 245fa foam improves the dimensional stability of the foam in both cold and hot humid environments. This is most evident in the hot humid environment. In fact the blends perform better than either of the pure compounds. This is an unexpected result.

**Table 17 - Foam Dimensional Stability**

| | 245fa | 245fa/ 1336mzzm 70/30 mole % | 245fa/ 1336mzzm 30/70 mole % | 1336mzzm |
|---|---|---|---|---|
| Dimensional Stability, vol.% changed | | | | |
| Cold, -29°C | | | | |
| 1 Day | -0.066 | -0.245 | -0.236 | -0.001 |
| 7 Day | 1.090 | -0.111 | 0.010 | 0.608 |
| 14 Day | 0.033 | -0.229 | -0.161 | -0.108 |
| | | | | |

| Hot/Humid, 70°C/95%R.H. | | | | |
|---|---|---|---|---|
| 1 Day | 7.388 | 3.657 | 4.899 | 5.326 |
| 7 Day | 21.617 | 14.060 | 16.385 | 17.720 |
| 14 Day | 30.616 | 22.013 | 26.563 | 28.501 |
| Thermal Conductivity | | | | |

Initially, foam produced with Z-HFO-1336mzzm and the 70/30 mole % Z-HFO-1336mzzm/245fa blend show the "hockey stick" curve shape traditionally found with high boiling blowing agents. This is attributed to the condensation of the blowing agent in the foam matrix at temperatures below the boiling point of the blowing agent. It is unanticipated that the 30/70 mole % Z-HFO-1336mzzm/245fa blend does not show the same curve shape since this is not an azeotropic composition. In addition the thermal conductivity of foams prepared with this blend is equivalent or slightly improved over those made with 245fa. The foam prepared of the 70/30 mole % 245fa/ Z-HFO-1336mzzm blend age slower than the 245fa and the Z-HFO-1336mzzm foams.

**Table 18 - Foam Thermal Conductivity**

| | 245fa | 245fa/ 1336mzzm 70/30 mole % | 245 fa/ 1336mzzm 30/70 mole % | 1336mzzm |
|---|---|---|---|---|
| Initial | | | | |
| 40 °F | 0.1318 | 0.1319 | 0.1347 | 0.1356 |
| 75 °F | 0.1481 | 0.1476 | 0.1452 | 0.1432 |
| 110 °F | 0.1660 | 0.1663 | 0.1629 | 0.1612 |

| 8 Day | | | | |
|---|---|---|---|---|
| 40 °F | 0.1381 | 0.1365 | 0.1380 | 0.1397 |
| 75 °F | 0.1555 | 0.1508 | 0.1493 | 0.1472 |
| 110 °F | 0.1742 | 0.1695 | 0.1692 | 0.1672 |

| 14 Day | | | | |
|---|---|---|---|---|
| 40 °F | 0.1409 | 0.1376 | 0.1391 | 0.1421 |
| 75 °F | 0.1585 | 0.1528 | 0.1524 | 0.1479 |
| 110 °F | 0.1774 | 0.1716 | 0.1740 | 0.1667 |

### METHODS AND SYSTEMS

Table 1 describes compositions of this invention which comprise, or consist essentially of Z-HFO-1336mzzm. These compositions are useful in connection with numerous methods and systems, including as heat transfer fluids in methods and systems for transferring heat, such as refrigerants used in refrigeration, air conditioning, including vehicle air conditioning systems, and heat pump systems. The compositions of this invention are also advantageous for in use in systems and methods of generating aerosols, preferably comprising or consisting of the aerosol propellant in such systems and methods. Methods of forming foams and methods of extinguishing and suppressing fire are also included as embodiments of this invention. The present invention also provides in certain aspects methods of removing residue from articles in which the present compositions are used as solvent compositions in such methods and systems.

### HEAT TRANSFER METHODS

The preferred heat transfer methods generally comprise providing a composition comprising, or consisting essentially of Z-HFO-1336mzzm, particularly blends as described in Table 1, and causing heat to be transferred to or from the composition changing the phase of the composition. For example, the present methods provide cooling by absorbing heat from a fluid or article, preferably by evaporating the present refrigerant composition in the vicinity of the body or fluid to be cooled to produce vapor comprising, or consisting essentially of, Z-HFO-1336mzzm.

For this use, the amount of the compound Z-HFO-1336mzzm in the composition of the invention can be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use of the composition of the invention.

Preferably the methods include the further step of compressing the refrigerant vapor, usually with a compressor or similar equipment to produce vapor of the present composition at a relatively elevated pressure. Generally, the step of compressing the vapor results in the addition of heat to the vapor, thus causing an increase in the temperature of the relatively high-pressure vapor. Preferably, the present methods include removing from this relatively high temperature, high pressure vapor at least a portion of the heat added by the evaporation and compression steps. The heat removal step preferably includes condensing the high temperature, high-pressure vapor while the vapor is in a relatively high-pressure condition to produce a relatively high-pressure liquid comprising, or consisting essentially of, Z-HFO-1336mzzm. This relatively high-pressure liquid preferably then undergoes a nominally isoenthalpic reduction in pressure to produce a relatively low temperature, low-pressure liquid. In such embodiments, it is this reduced temperature refrigerant liquid which is then vaporized by heat transferred from the body or fluid to be cooled.

In another process embodiment of the invention, the compositions of the invention may be used in a method for producing heating which comprises condensing a refrigerant comprising, or consisting essentially of, Z-HFO-1336mzzm, particularly blends as described in Table 1, in the vicinity of a liquid or body to be heated. Such methods, as mentioned hereinbefore, frequently are reverse cycles to the refrigeration cycle described above.

For this use, the amount of the compound Z-HFO-1336mzzm in the composition of the invention can be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use of the composition of the invention.

### REFRIGERANT COMPOSITIONS

The present methods, systems and compositions comprising, or consisting essentially of Z-HFO-1336mzzm, and in particular, blends as described in Table 1, are thus adaptable for use in connection with automotive air conditioning systems and devices, commercial refrigeration systems and devices, chillers, residential refrigerator and freezers, general air conditioning systems, heat pumps, and the like.

Many existing refrigeration systems are currently adapted for use in connection with existing refrigerants, and the compositions of the present invention are believed to be adaptable for use in many of such systems, either with or without system modification. In many applications the compositions of the present invention may provide an advantage as a replacement in systems, which are currently based on refrigerants having a relatively high capacity. Furthermore, in embodiments where it is desired to use a lower capacity refrigerant composition of the present invention, for reasons of efficiency for example, to replace a refrigerant of higher capacity, such embodiments of the present compositions provide a potential advantage. Thus, it is preferred in certain embodiments to use compositions comprising, or consisting essentially of, Z-HFO-1336mzzm, either alone or in combination with one or more other compounds, particularly blends as described in Table 1, as a replacement for existing refrigerants, such as HCFC-123 or HFC-134a. In certain applications, the refrigerants of the present invention potentially permit the beneficial use of larger displacement compressors, thereby resulting in better energy efficiency than other refrigerants, such as HCFC-123 or HFC-134a. Therefore the refrigerant compositions of the present invention, particularly compositions comprising, or consisting essentially of, Z-HFO-1336mzzm, provide the possibility of achieving a competitive advantage on an energy basis for refrigerant replacement applications.

Although, as described above, it is contemplated that the compositions of the present invention may include the compounds of the present invention in widely ranging amounts, it is generally preferred that refrigerant compositions of the present invention comprise Z-HFO-1336mzzm, in an amount that is at least about 50% by weight, and even more preferably at least about 70% by weight, of the composition.

The compositions of the present invention may include other components for the purpose of enhancing or providing certain functionality to the composition, or in some cases to reduce the cost of the composition. For example, refrigerant compositions according to the present invention, especially those used in vapor compression systems, include a lubricant, generally in amounts of from about 30 to about 50 percent by weight of the composition. Furthermore, the present compositions may also include a compatibilizer, such as propane, for the purpose of aiding compatibility and/or solubility of the lubricant. Such compatibilizers, including propane, butanes and pentanes, are preferably present in amounts of from about 0.5 to about 5 percent by weight of the composition.

Combinations of surfactants and solubilizing agents may also be added to the present compositions to aid oil solubility, as disclosed by U.S. Pat. No. 6,516,837, the disclosure of which is incorporated by reference. Commonly used refrigeration lubricants such as Polyol Esters (POEs) and Poly Alkylene Glycols (PAGs), silicone oil, mineral oil, alkyl benzenes (ABs) and poly(alpha-olefin) (PAO) that are used in refrigeration machinery with hydrofluorocarbon (HFC) refrigerants may be used with the refrigerant compositions of the present invention.

It is contemplated that the compositions of the present, including particularly those comprising, or consisting essentially of, Z-HFO-1336mzzm, and particularly blends as set forth in Table 1, also have advantage (either in original systems or when used as a replacement for refrigerants such as R-12 and R-500), in chillers typically used in connection with commercial air conditioning systems. In certain of such embodiments it is preferred to including in the present Z-HFO-1336mzzm compositions from about 0.5 to about 5% of a flammability suppressant, such as CF3I.

In certain preferred embodiments, the compositions of the present invention further comprise a lubricant. Any of a variety of conventional lubricants may be used in the compositions of the present invention. An important requirement for the lubricant is that, when in use in a refrigerant system, there must be sufficient lubricant returning to the compressor of the system such that the compressor is lubricated. Thus, suitability of a lubricant for any given system is determined partly by the refrigerant/lubricant characteristics and partly by the characteristics of the system in which it is intended to be used. Examples of suitable lubricants include mineral oil, alkyl benzenes, polyol esters, including polyalkylene glycols, PAG oil, and the like. Mineral oil, which comprises paraffin oil or naphthenic oil, is commercially available. Commercially available mineral oils include Witco LP 250 (registered trademark) from Witco, Zerol 300 (registered trademark) from Shrieve Chemical, Sunisco 3GS from Witco, and Calumet R015 from Calumet. Commercially available alkyl benzene lubricants include Zerol 150 (registered trademark). Commercially available esters include neopentyl glycol dipelargonate which is available as Emery 2917 (registered trademark) and Hatcol 2370 (registered trademark). Other useful esters include phosphate esters, dibasic acid esters, and fluoroesters. Preferred lubricants include polyalkylene glycols and esters. Certain more preferred lubricants include polyalkylene glycols.

Any of a wide range of methods for introducing the present refrigerant compositions to a refrigeration system can be used in the present invention. For example, one method comprises attaching a refrigerant container to the low-pressure side of a refrigeration system and turning on the refrigeration system compressor to pull the refrigerant into the system. In such embodiments, the refrigerant container may be placed on a scale such that the amount of refrigerant composition entering the system can be monitored. When a desired amount of refrigerant composition has been introduced into the system, charging is stopped. Alternatively, a wide range of charging tools, known to those of skill in the art, is commercially available. Accordingly, in light of the above disclosure, those of skill in the art will be readily able to introduce the refrigerant compositions of the present invention into refrigeration systems according to the present invention without undue experimentation.

### REFRIGERANT EXAMPLE

This example demonstrates the use of the compositions of the present invention for use as a refrigerant composition.

The fluid of choice for centrifugal chillers is 2,2-dichloro-1,1,1-trifluoroethane (R123). Due to the ozone depletion potential of R123 it is currently banned under the Montreal protocol. It is important to maintain the high coefficient of performance (COP) when finding a replacement fluid for R123. A single stage chiller consists if a compressor which pressurizes a low pressure gas and delivers it to the evaporator. The high pressure fluid is then condensed at a relatively high temperature, for this case the condenser is maintained at 40°C. The condensed fluid is then passed through an expansion device which lowers both the temperature and pressure of the fluid and is introduced into the evaporator; in this case the evaporator is maintained at 2°C. The cold low pressure gas is then used to transfer heat away from the body which requires cooling by evaporating the fluid in the evaporator. The thermodynamic performance of a refrigerant can be calculated using standard refrigerant cycle analysis techniques outlined in thermodynamic texts such as R.C. Downing, Fluorocarbon Refrigerants Handbook, Chapter 3, Prentice-Hall. 1988. The COP of a single compressor chiller was determined at a condenser temperature of 2°C, evaporator temperature of 40°C, and a compressor efficiency of 0.75. The COPs of HCFC-123 and Z-HFO-1336mzzm in a single compressor system are both 4.6. The ability to maintain a COP equal to that of HCFC-123 while being non-ozone depleting and having very low GWP makes Z-HFO-1336mzzm a very fluid for a centrifugal chiller.

### POWER CYCLE USE

Rankine cycle systems are known to be a simple and reliable means to convert heat energy into mechanical shaft power. Organic working fluids are useful in place of water/steam when low-grade thermal energy is encountered. Water/steam systems operating with low-grade thermal energy (typically 400°F and lower) will have associated high volumes and low pressures. To keep system size small and efficiency high, organic working fluids with boiling points near room temperature are employed. Such fluids would have higher gas densities lending to higher capacity and favorable transport and heat transfer properties lending to higher efficiency as compared to water at low operating temperatures. In industrial settings there arc more opportunities to use flammable working fluids such as toluene and pentane, particularly when the industrial setting has large quantities of flammables already on site in processes or storage. For instances where the risk associated with use of a flammable working fluid is not acceptable, such as power generation in populous areas or near buildings, other fluids such as CFC-113 and CFC-11 were used. Although these materials were non-flammable, they were a risk to the environment because of their ozone-depletion potential. Ideally, the organic working fluid should be environmentally acceptable, non-flammable, of a low order of toxicity, and operate at positive pressures.

Organic Rankine Cycle (ORC) systems are often used to recover waste heat from industrial processes. In combined heat and power (cogeneration) applications, waste heat from combustion of fuel used to drive the prime mover of a generator set is recovered and used to make hot water for building heat, for example, or for supplying heat to operate an absorption chiller to provide cooling. In some cases, the demand for hot water is small or does not exist. The most difficult case is when the thermal requirement is variable and load matching becomes difficult, confounding efficient operation of the combined heat and power system. In such an instance, it is more useful to convert the waste heat to shaft power by using an organic Rankine cycle system. The shaft power can be used to operate pumps, for example, or it may be used to generate electricity. By using this approach, the overall system efficiency is higher and fuel utilization is greater. Air emissions from fuel combustion can be decreased since more electric power can be generated for the same amount of fuel input.

The process that produces waste heat is at least one selected from the group consisting of fuel cells, internal combustion engines, internal compression engines, external combustion engines, and turbines. Other sources of waste heat can be found in association with operations at oil refineries, petrochemical plants, oil and gas pipelines, chemical industry, commercial buildings, hotels, shopping malls, supermarkets, bakeries, food processing industries, restaurants, paint curing ovens, furniture making, plastics molders, cement kilns, lumber kilns (drying), calcining operations, steel industry, glass industry, foundries, smelting, air-conditioning, refrigeration, and central heating. See U.S. Patent No. 7,428,816, the disclosure of which is hereby incorporated herein by reference.

Preferred compositions for ORC power cycle use are described below in Table 19 (with all percentages being in percent by weight and being understood to be proceeded by the word "about").

**Table 19 - ORC Blends**

| Compound mixed with Z-HFO-1336mzzm | | Preferred Ranges wt % | More Preferred Ranges wt % | Most Preferred Ranges wt % |
|---|---|---|---|---|
| HFOS | | | | |
| | cis-HFO-1234ze | 1 to 99 | 1 to 70 | 1 to 50 |
| | HFO-1234yf | 1 to 99 | 1 to 70 | 1 to 50 |
| | HFO 1225ycZ | 1 to 99 | 1 to 70 | 1 to 50 |
| | HFO 1225yeE | 1 to 99 | 1 to 70 | 1 to 50 |
| | HFO1225yc | 1 to 99 | 1 to 70 | 1 to 50 |
| | HFO-1233zd | 1 to 99 | 20 to 80 | 30 to 70 |
| | HFC-1233xf | 1 to 99 | 20 to 80 | 30 to 70 |
| | CF3CH=CHCF3 (E) | 1 to 99 | 1 to 70 | 1 to 50 |
| | (CF3)2CFCH=CHF (E&Z) | 1 to 99 | 1 to 70 | 1 to 50 |
| | (CF3)2CFCH=CF2 | 1 to 99 | 1 to 70 | 1 to 50 |
| | CF3CHFC=CHF (E & Z) | 1 to 99 | 1 to 70 | 1 to 50 |
| | (C2F5)(CF3)C=CH2 | 1 to 99 | 1 to 70 | 1 to 50 |
| HFCs | | | | |
| | HFC-245fa | 1 to 99 | 1 to 70 | 1 to 25 |
| | HFC-245eb | 1 to 99 | 1 to 70 | 1 to 25 |
| | HFC-245ca | 1 to 99 | 1 to 70 | 1 to 30 |
| | HFC-227ea | 1 to 99 | 1 to 70 | 1 to 10 |
| | HFC-236ea | 1 to 99 | 1 to 70 | 1 to 20 |
| | HFC-236fa | 1 to 99 | 1 to 70 | 1 to 5 |
| | HFC-365mfc | 1 to 99 | 1 to 70 | 1 to 25 |
| | HFC-43-10mee | 1 to 99 | 1 to 70 | 1 to 15 |
| HFEs | | | | |
| | CHF2-O-CHF2 | 1 to 99 | 1 to 70 | 1 to 50 |
| | CHF2-O-CH2F | 1 to 99 | 1 to 70 | 1 to 50 |
| | CH2F-O-CH2F | 1 to 99 | 1 to 70 | 1 to 50 |
| | CH2F-O-CH3 | 1 to 99 | 1 to 70 | 1 to 50 |
| | CYCLO-CF2-CH2-CF2-O | 1 to 99 | 1 to 70 | 1 to 50 |
| | CYCLO-CF2-CF2-CH2-O | 1 to 99 | 1 to 70 | 1 to 50 |
| | CHF2-O-CF2-CHF2 | 1 to 99 | 1 to 70 | 1 to 50 |
| | CF3-CF2-O-CH2F | 1 to 99 | 1 to 70 | 1 to 50 |
| | CHF2-O-CHF-CF3 | 1 to 99 | 1 to 70 | 1 to 50 |
| | CHF2-O-CF2-CHF2 | 1 to 99 | 1 to 70 | 1 to 50 |
| | CH2F-O-CF2-CHF2 | 1 to 99 | 1 to 70 | 1 to 50 |
| | CF3-O-CF2-CH3 | 1 to 99 | 1 to 70 | 1 to 50 |
| | CHF2-CHF-O-CHF2 | 1 to 99 | 1 to 70 | 1 to 50 |
| | CF3-O-CHF-CH2F | 1 to 99 | 1 to 70 | 1 to 50 |
| | CF3-CHF-O-CH2F | 1 to 99 | 1 to 70 | 1 to 50 |
| | CF3-O-CH2-CHF2 | 1 to 99 | 1 to 70 | 1 to 50 |
| | CHF2-O-CH2-CF3 | 1 to 99 | 1 to 70 | 1 to 50 |
| | CH2F-CF2-O-CH2F | 1 to 99 | 1 to 70 | 1 to 50 |
| | CHF2-O-CF2-CH3 | 1 to 99 | 1 to 70 | 1 to 50 |
| | CHF2-CF2-O-CH3 (254pc) | 1 to 99 | 1 to 70 | 1 to 50 |
| | CH2F-O-CHF-CH2F | 1 to 99 | 1 to 70 | 1 to 50 |
| | CHF2-CHF-O-CH2F | 1 to 99 | 1 to 70 | 1 to 50 |
| | CF3-O-CHF-CH3 | 1 to 99 | 1 to 70 | 1 to 50 |
| | CF3-CHF-O-CH3 | 1 to 99 | 1 to 70 | 1 to 50 |
| | CHF2-O-CH2-CHF2 | 1 to 99 | 1 to 70 | 1 to 50 |
| | CF3-O-CH2-CH2F | 1 to 99 | 1 to 70 | 1 to 50 |
| | CF3-CH2-O-CH2F | 1 to 99 | 1 to 70 | 1 to 50 |
| | CF2H-CF2-CF2-O-CH3 | 1 to 99 | 1 to 70 | 1 to 50 |
| Hydrocarbons | | | | |
| | Propane | 1 to 99 | 20 to 95 | 40 to 95 |
| | Butane | 1 to 99 | 20 to 95 | 40 to 95 |
| | Isobutane | 1 to 99 | 20 to 95 | 40 to 95 |
| | n-pentane (high HFO) | 1 to 99 | 50 to 99 | 60 to 99 |
| | n-pentane (high n-pentane) | 1 to 99 | 1 to 30 | 1 to 20 |
| | Isopentane (High HFO) | 1 to 99 | 50 to 99 | 60 to 99 |
| | Isopentane (High isopentane) | 1 to 99 | 1 to 30 | 1 to 20 |
| | Neopentane (High HFO) | 1 to 99 | 50 to 99 | 60 to 99 |
| | Neopentane (High neopentane) | 1 to 99 | 1 to 30 | 1 to 20 |
| | Cyclopentane (High HFO) | 1 to 99 | 50 to 99 | 60 to 99 |
| | Cyclopentane (High cyclopentane) | 1 to 99 | 1 to 30 | 1 to 20 |
| | n-hexane | 1 to 99 | 20 to 95 | 40 to 95 |
| | Isohexane | 1 to 99 | 20 to 95 | 40 to 95 |
| | Heptane | 1 to 99 | 20 to 95 | 40 to 95 |
| Others | | | | |
| | Trans-1,2 dichloroethylene | 1 to 99 | 1 to 50 | 1 to 30 |
| | Mixtures with cis-HFO-1234ze + HFC-245fa | 1 to 25 / 1 to 50 | 1 to 20 / 1 to 25 | 1 to 15 / 1 to 10 |

One specific embodiment of a power cycle use of this compound is a process for recovering waste heat in an Organic Rankine Cycle system in which the working fluid is a composition comprising, or consisting essentially of, Z-HFO-1336mzzm and optionally, one or more additional compounds, as set forth above in Table 19.

### POWER CYCLE EXAMPLE

Following the procedure outlined in Smith, J. M. et al., Introduction to Chemical Engineering Thermodynamics; McGraw-Hill (1996), the effectiveness of various working fluids in an organic Rankine cycle can by compared. The conditions used in the organic Rankine cycle calculations in this example are a pump efficiency of 75%, expander efficiency of 80%, boiler temperature of 190°C, condenser temperature of 45°C and 1000 W of heat supplied to the boiler. The performance of Z-HFO-1336mzzm is compared to the commercially available fluid HFC-245fa (available from Honeywell). The thermal efficiency of HFC-245fa and Z-HFO-1336mzzm at the conditions specified is 0.142 and 0.145, respectively. This shows that Z-HFO-1336mzzm. Z-HFO-1336mzzm is also non-flammable and has a low global warming potential.

This example demonstrates the use of the compositions of the present invention for use as a Rankine power cycle composition. It has been found that using the compound Z-HFO-1336mzzm as the power cycle fluid in an organic Rankine cycle, thermal efficiency is increased by about 2% over a similar cycle using HFC-245fa as the power cycle fluid.

### CLEANING AND CONTAMINANT REMOVAL

The present invention also provides methods of removing containments from a product, part, component, substrate, or any other article or portion thereof by applying to the article a composition of the present invention comprising, or consisting essentially of, Z-HFO-1336mzzm, and in particular, the blends set forth in Table 1. For the purposes of convenience, the term "article" is used herein to refer to all such products, parts, components, substrates, and the like and is further intended to refer to any surface or portion thereof. Furthermore, the term "contaminant" is intended to refer to any unwanted material or substance present on the article, even if such substance is placed on the article intentionally. For example, in the manufacture of semiconductor devices it is common to deposit a photoresist material onto a substrate to form a mask for the etching operation and to subsequently remove the photoresist material from the substrate. The term "contaminant" as used herein is intended to cover and encompass such a photo resist material.

For this use, the amount of the compound Z-HFO-1336mzzm in the composition of the invention can be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use of the composition of the invention.

Preferred methods of the present invention comprise applying the present composition to the article. Although it is contemplated that numerous and varied cleaning techniques can employ the compositions of the present invention to good advantage, it is considered to be particularly advantageous to use the present compositions in connection with supercritical cleaning techniques. Supercritical cleaning is disclosed in U.S. Pat. No. 6,589,355, which is incorporated herein by reference.

For supercritical cleaning applications, it is preferred in certain embodiments to include in the present cleaning compositions, in addition to the composition of the present invention, another component, such as CO2 and other additional components known for use in connection with supercritical cleaning applications.

It may also be possible and desirable in certain embodiments to use the present cleaning compositions in connection with particular sub-critical vapor degreasing and solvent cleaning methods. For all solvent uses, compositions containing the compound Z-HFO-1336mzzm may preferably be blended with one or more of the following compounds; cis-1234ze, cis-1233zd, HFC-245fa, Methylal (dimethoxymethane), methylethylketone, methylisobutylketone, and/or HFC-134a. More preferred blends comprise Z-HFO-1336mzzm blended with one or more of the following compounds; pentanes, hexanes, HFC-365, C₄F₉-O-CH₃, C₄F₉-O-C₂H₅, propane, butane, isobutane, and/or dimethylether. Most preferred blends comprise Z-HFO-1336mzzm blended with one or more of the following compounds; trans-1,2-dichloroethylene, trans-1234ze, trans-1233zd, trans-1336, HFC-43-10, HFC-152a, methanol, ethanol, isopropanol, and/or acetone.

Another cleaning embodiment of the invention comprises the removal of contaminants from vapor compression systems and their ancillary components when these systems are manufactured and serviced. As used herein, the term "contaminants" refers to processing fluids, lubricants, particulates, sludge, and/or other materials that are used in the manufacture of these systems or generated during their use. In general, these contaminants comprise compounds such as alkylbenzenes, mineral oils, esters, polyalkyleneglycols, polyvinylethers and other compounds that arc made primarily of carbon, hydrogen and oxygen. The compositions of the present invention will be useful for this purpose.

### CLEANING COMPOSITION EXAMPLE

This example demonstrates the use of the compositions of the present invention comprising, or consisting essentially of Z-HFO-1336mzzm, and in particular, blends as described in Table 1, for use as a cleaning composition. For this use, the amount of the compound Z-HFO-1336mzzm in the composition of the invention can be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use of the composition of the invention.

Mixtures are prepared containing 70% by weight Z-HFO-1336mzzm with about 30% by weight trans-1,2 dichloroethylene. Several stainless steel coupons are soiled with mineral oil, rosin flux or other contaminants. Then these coupons are then immersed in the solvent blend. The blend could remove the oils in a short period of time. The coupons are observed visually for cleanliness. Similar results are expected with the other mixtures. Similar results are also expected with silicon oil.

### PROPELLANTS FOR SPRAYABLE COMPOSITIONS

In another embodiment, the compositions of this invention comprising, or consisting essentially of, Z-HFO-1336mzzm, and in particular, blends as described in Table 1, may be used as propellants in sprayable compositions, either alone or in combination with known propellants. For this use, the amount of the compound Z-HFO-1336mzzm in the composition of the invention can be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use of the composition of the invention.

The sprayable composition includes a material to be sprayed and a propellant comprising, or consisting essentially of Z-HFO-1336mzzm, and in particular, blends as described in Table 1. Inert ingredients, solvents, and other materials may also be present in the sprayable mixture. Preferably, the sprayable composition is an aerosol. Suitable materials to be sprayed include, without limitation, cosmetic materials such as deodorants, perfumes, hair sprays, cleansers, and polishing agents as well as medicinal materials such as anti-asthma and anti-halitosis medications.

For aerosol uses, compositions containing the compound cis-1,1,1,4,4,4-hexafluoro-2-butene (Z-HFO-1336mzzm) may preferably be blended with one or more of the following compounds; cis-1234ze, cis-1233zd, HFC-245fa, Methylal (dimethoxymethane), methylethylketone, methylisobutylketone, and/or HFC-134a. More preferred blends comprise Z-HFO-1336mzzm blended with one or more of the following compounds; pentanes, hexanes, HFC-365, C₄F₉-O-CH₃, C₄F₉-O-C₂H₅, propane, butane, isobutane, and/or dimethylether. Most preferred blends comprise Z-HFO-1336mzzm blended with one or more of the following compounds; trans-1,2-dichloroethylene, trans-1234ze, trans-1233zd, trans-1336, HFC-43-10, HFC-152a, methanol, ethanol, isopropanol, and/or acetone.

In this use, the active ingredient to be sprayed is mixed with inert ingredients, solvents, and other materials may also be present in the sprayable mixture. Preferably, the sprayable composition is an aerosol. Suitable active materials to be sprayed include, without limitation, lubricants, insecticides, cleaners, cosmetic materials such as deodorants, perfumes and hair sprays, polishing agents, as well as medicinal materials such as skin cooling agents (sunburn treatment), topical anesthetics and anti-asthma medications.

In another aspect, the present invention provides propellant comprising, or consisting essentially of, Z-HFO-1336mzzm, either alone or in combination with one or more other compounds, in particular blends as set forth in Table 1, such propellant composition preferably being a sprayable composition. The propellant compositions of the present invention preferably comprise a material to be sprayed and a propellant comprising, or consisting essentially of, Z-HFO-1336mzzm. Inert ingredients, solvents, and other materials may also be present in the sprayable mixture. Preferably, the sprayable composition is an aerosol. Suitable materials to be sprayed include, without limitation, lubricants, insecticides, cleaners, cosmetic materials such as deodorants, perfumes and hair sprays, polishing agents as well as medicinal materials such as anti-asthma components, and any other medication or the like, including preferably any other medicament or agent intended to be inhaled. The medicament or other therapeutic agent is preferably present in the composition in a therapeutic amount, with a substantial portion of the balance of the composition comprising, or consisting essentially of, Z-HFO-1336mzzm.

Aerosol products for industrial, consumer or medical use typically contain one or more propellants along with one or more active ingredients, inert ingredients or solvents. The propellant provides the force that expels the product in aerosolized form. While some aerosol products are propelled with compressed gases like carbon dioxide, nitrogen, nitrous oxide and even air, most commercial aerosols use liquefied gas propellants. The most commonly used liquefied gas propellants are hydrocarbons such as butane, isobutane, and propane. Dimethyl ether and HFC-152a (1,1-difluoroethane) are also used, either alone or in blends with the hydrocarbon propellants. Unfortunately, all of these liquefied gas propellants arc highly flammable and their incorporation into aerosol formulations will often result in flammable aerosol products.

Applicants have come to appreciate the continuing need for nonflammable, liquefied gas propellants with which to formulate aerosol products. The present invention provides compositions of the present invention, particularly and preferably compositions comprising, or consisting essentially of, Z-HFO-1336mzzm, and in particular, those blends set forth in Table 1, for use in certain industrial aerosol products, including for example spray cleaners, lubricants, and the like, and in medicinal aerosols, including for example to deliver medications to the lungs or mucosal membranes. Examples of this includes metered dose inhalers (MDIs) for the treatment of asthma and other chronic obstructive pulmonary diseases and for delivery of medicaments to accessible mucous membranes or intranasally. The present invention thus includes methods for treating ailments, diseases and similar health related problems of an organism (such as a human or animal) comprising applying a composition of the present invention containing a medicament or other therapeutic component to the organism in need of treatment. In certain preferred embodiments, the step of applying the present composition comprises providing a MDI containing the composition of the present invention (for example, introducing the composition into the MDI) and then discharging the present composition from the MDI.

As used herein, the term "nonflammable" refers to compounds and compositions of the present invention which do not exhibit a flashpoint as measured by one of the standard flash point methods, for example ASTM-1310-86 "Flash point of liquids by tag Open-cup apparatus."

The present compositions can be used to formulate a variety of industrial aerosols or other sprayable compositions such as contact cleaners, dusters, lubricant sprays, and the like, and consumer aerosols such as personal care products, household products and automotive products. Z-HFO-1336mzzm is particularly preferred for use as an important component of propellant compositions for in medicinal aerosols such as metered dose inhalers. The medicinal aerosol and/or propellant and/or sprayable compositions of the present invention in many applications include, in addition to Z-HFO-1336mzzm, a medicament such as a beta-agonist, a corticosteroid or other medicament, and, optionally, other ingredients, such as surfactants, solvents, other propellants, flavorants and other excipients.

### STERILIZATION

Many articles, devices and materials, particularly for use in the medical field, must be sterilized prior to use for the health and safety reasons, such as the health and safety of patients and hospital staff. The present invention provides methods of sterilizing comprising contacting the articles, devices or material to be sterilized with a composition of the present invention comprising, or consisting essentially of, Z-HFO-1336mzzm, and in particular, the blends defined in Table 1, and optionally in combination with one or more additional sterilizing agents.

For this use, the amount of the compound Z-HFO-1336mzzm in the composition of the invention can be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use of the composition of the invention.

While many sterilizing agents are known in the art and are considered to be adaptable for use in connection with the present invention, in certain preferred embodiments sterilizing agent comprises ethylene oxide, formaldehyde, hydrogen peroxide, chlorine dioxide, ozone and combinations of these. In certain embodiments, ethylene oxide is the preferred sterilizing agent. Those skilled in the art, in view of the teachings contained herein, will be able to readily determine the relative proportions of sterilizing agent and the present compound(s) to be used in connection with the present sterilizing compositions and methods, and all such ranges are within the broad scope hereof.

As is known to those skilled in the art, certain sterilizing agents, such as ethylene oxide, are extremely flammable components, and the compound(s) in accordance with the present invention are included in the present compositions in amounts effective, together with other components present in the composition, to reduce the flammability of the sterilizing composition to acceptable levels. The sterilization methods of the present invention may be either high or low-temperature sterilization of the present invention involves the use of a compound or composition of the present invention at a temperature of from about 250°F to about 270°F, preferably in a substantially sealed chamber. The process can be completed usually in less than about two hours. However, some articles, such as plastic articles and electrical components, cannot withstand such high temperatures and require low-temperature sterilization.

### STERILIZATION EXAMPLES

In low temperature sterilization methods, the article to be sterilized is exposed to a fluid comprising, or consisting essentially of, Z-HFO-1336mzzm at a temperature of from about room temperature to about 200°F, more preferably at a temperature of from about room temperature to about 100°F.

The low-temperature sterilization of the present invention is preferably at least a two-step process performed in a substantially sealed, preferably air tight, chamber. In the first step (the sterilization step), the articles having been cleaned and wrapped in gas permeable bags are placed in the chamber.

Air is then evacuated from the chamber by pulling a vacuum and perhaps by displacing the air with steam. In certain embodiments, it is preferable to inject steam into the chamber to achieve a relative humidity that ranges preferably from about 30% to about 70%. Such humidities may maximize the sterilizing effectiveness of the sterilant, which is introduced into the chamber after the desired relative humidity is achieved. After a period of time sufficient for the sterilant to permeate the wrapping and reach the interstices of the article, the sterilant and steam are evacuated from the chamber.

In the preferred second step of the process (the aeration step), the articles are aerated to remove sterilant residues. Removing such residues is particularly important in the case of toxic sterilants, although it is optional in those cases in which the substantially non-toxic compounds of the present invention are used. Typical aeration processes include air washes, continuous aeration, and a combination of the two. An air wash is a batch process and usually comprises evacuating the chamber for a relatively short period, for example, 12 minutes, and then introducing air at atmospheric pressure or higher into the chamber.

As used herein the term "non-toxic" refers to compounds and compositions of the present invention which have an acute toxicity level substantially less than, and preferably at least about 30 relative percent less than, the toxicity level of HFO-1223xd, as measured by the method published in Anesthesiology, Vol. 14, pp. 466-472, 1953, incorporated here by reference.

This cycle is repeated any number of times until the desired removal of sterilant is achieved. Continuous aeration typically involves introducing air through an inlet at one side of the chamber and then drawing it out through an outlet on the other side of the chamber by applying a slight vacuum to the outlet. Frequently, the two approaches are combined. For example, a common approach involves performing air washes and then an aeration cycle.

### LUBRICANTS

In certain preferred embodiments, the compositions of the present invention comprising, or consisting essentially of, Z-HFO-1336mzzm, and in particular, the blends defined in Table 1, may further comprise a lubricant. Any of a variety of conventional lubricants may be used in the compositions of the present invention. An important requirement for the lubricant is that, when in use in a refrigerant system, there must be sufficient lubricant returning to the compressor of the system such that the compressor is lubricated. Thus, suitability of a lubricant for any given system is determined partly by the refrigerant/lubricant characteristics and partly by the characteristics of the system in which it is intended to be used.

Examples of suitable lubricants include mineral oil, alkyl benzenes, polyol esters, including polyalkylene glycols, PAG oil, and the like. Mineral oil, which comprises paraffin oil or naphthenic oil, is commercially available. Commercially available mineral oils include Witco LP 250 (registered trademark) from Witco, Zerol 300 (registered trademark) from Shrieve Chemical, Sunisco 3GS from Witco, and Calumet R015 from Calumet. Commercially available alkyl benzene lubricants include Zerol 150 (registered trademark). Commercially available esters include neopentyl glycol dipelargonate which is available as Emery 2917 (registered trademark) and Hatcol 2370 (registered trademark). Other useful esters include phosphate esters, dibasic acid esters, and fluoroesters. Preferred lubricants include polyalkylene glycols and esters. Certain more preferred lubricants include polyalkylene glycols.

### EXTRACTION OF FLAVORS AND FRAGRANCES

The compositions of the present invention comprising, or consisting essentially of Z-HFO-1336mzzm, and in particular, the blends as described in Table 1, also provide advantage when used to carry, extract or separate desirable materials from biomass. These materials include, but are not limited to, essential oils such as flavors and fragrances, oils which may be used as fuel, medicinals, nutraceuticals, etc.

For this use, the amount of the compound Z-HFO-1336mzzm in the composition of the invention can be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use of the composition of the invention.

### EXTRACTION EXAMPLE

The suitability of the present compositions for this purpose is demonstrated by a test procedure in which a sample of Jasmone is put into a heavy walled glass tube. A suitable amount of a Z-HFO-1336mzzm containing composition of the present invention is added to the glass tube. The tube is then frozen and sealed. Upon thawing the tube, when the mixture has one liquid phase containing Jasome and the Z-HFO-1336mzzm containing composition of this invention; this test establishes the favorable use of the composition as an extractant, carrier or part of delivery system for flavor and fragrance formulations, in aerosol and other formulations. It also establishes its potential as an extractant of flavors and fragrances, including from plant matter.

### FLAMMABILITY REDUCTION METHODS

According to certain other preferred embodiments, the present invention provides methods for reducing the flammability of fluids, said methods comprising adding a Z-HFO-1336mzzm containing composition, such as the blends defined in Table 1, to said fluid. The flammability associated with any of a wide range of otherwise flammable fluids may be reduced according to the present invention. For example, the flammability associated with fluids such as ethylene oxide, flammable hydrofluorocarbons and hydrocarbons, including: HFC-152a, 1,1,1-trifluoroethane (HFC-143a), difluoromethane (HFC-32), propane, hexane, octane, and the like can be reduced according to the present invention. For the purposes of the present invention, a flammable fluid may be any fluid exhibiting flammability ranges in air as measured via any standard conventional test method, such as ASTM E-681, and the like.

For this use, the amount of the compound Z-HFO-1336mzzm in the composition of the invention can be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use of the composition of the invention.

Any suitable amounts of the present compounds or compositions may be added to reduce flammability of a fluid according to the present invention. As will be recognized by those of skill in the art, the amount added will depend, at least in part, on the degree to which the subject fluid is flammable and the degree to which it is desired to reduce the flammability thereof. In certain preferred embodiments, the amount of compound or composition added to the flammable fluid is effective to render the resulting fluid substantially non-flammable.

### FLAMMABILITY REDUCTION EXAMPLE

This example demonstrates the use of the compositions of the present invention for reduction of flammability of another composition.

In an ASTM E681 apparatus at ambient conditions, one mixes isopentane vapor and Z-HFO-1336mzzm to find that the lower flammability limit (LFL) increases as more Z-HFO-1336mzzm is added. This indicates lower flammability for the blend than that of the isopentane by itself leading; a less flammable material which is easier to use safely. This higher LFL allow higher concentration in air without concern for ignition source and potential fires or explosions.

Two aerosol cans are filled with methanol/water and one is pressurized with HFC-152a while the other is pressurized with HFC-152a/ Z-HFO-1336mzzm. When the aerosols from the cans are sprayed over and into a candle flame as in the aerosol flame extension test procedure one observe less flame extension from the can that was pressured with Z-HFO-1336mzzm.

### FLAME SUPPRESSION METHODS

The present invention further provides methods of suppressing a flame, said methods comprising contacting a flame with a Z-HFO-1336mzzm containing composition of the present invention, particularly the blends described in Table 1. If desired, additional flame suppressing agents can also be used with the composition of the present invention, either in admixture, or as a secondary flame suppressing agent. One class of compounds for this purpose is the fluoroketones. One especially preferred fluoroketone is dodecafluoro-2-methylpentan-3-one, which is sold by the 3M Company under the trade name Novec 1230.

For this use, the amount of the compound Z-HFO-1336mzzm in the composition of the invention can be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use of the composition of the invention.

Any suitable methods for contacting the flame with the present composition may be used. For example, a composition of the present invention may be sprayed, poured, and the like onto the flame, or at least a portion of the flame may be immersed in the composition.

### FLAME SUPPRESSION EXAMPLE

This example demonstrates the use of the compositions comprising, or consisting essentially of Z-HFO-1336mzzm, and in particular, the blends as described in Table 1, for use as a flame suppression composition.

For this use, the amount of the compound Z-HFO-1336mzzm in the composition of the invention can be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use of the composition of the invention.

To evaluate total flooding fire suppression applications the NFPA 2001 cup burner is typically used. Here a small fire of heptane is located in a chimney which has air flowing around the flame to supply the needed oxygen. To this air stream Z-HFO-1336mzzm is added until the flame is extinguished. The concentration obtain thusly with appropriate safety factor as outlined in NFPA 2001 can be used to extinguish fires.

Fires can be extinguished locally using portable fire extinguishers. Such applications are classified as streaming applications. Using UL 711 a wood crib fire is started and extinguished using Z-HFO-1336mzzm. Secondly a heptane pan fire is tested using Z-HFO-1336mzzm. The results of this UL 711 testing give one the rating for the fire extinguisher tested.

While the present invention has been particularly shown and described with reference to preferred embodiments, it will be readily appreciated by those of ordinary skill in the art that various changes and modifications may be made without departing from the scope of the invention. It is intended that the claims be interpreted to cover the disclosed embodiment, those alternatives which have been discussed above and all equivalents thereto.
The present invention will now be further described by reference to the following numbered embodiments.
1. A mixture comprising the compound cis-1,1,1,4,4,4-hexafluoro-2-butene and at least one additional compound selected from the group consisting of HFOs, HFCs, HFEs, CFCs, CO2, olefins, organic acids, alcohols, hydrocarbons, ethers, aldehydes, ketones, and others such as methyl formate, formic acid, trans-1,2 dichloroethylene, carbon dioxide, cis-HFO-1234ze + HFO-1225yez; mixtures of these plus water; mixtures of these plus CO2; mixtures of these trans 1,2-dichloroethylene (DCE); mixtures of these plus methyl formate; mixtures with cis-HFO-1234ze + CO2; mixtures with cis-HFO-1234ze + HFO-1225yez + CO2; and mixtures with cis-HFO-1234ze + HFC-245fa.
2. The mixture of Embodiment 1, wherein the additional compound comprises one or more of the compounds selected from the group consisting of trans-1,2-dichloroethylene; carbon dioxide; cis-HFO-1234ze; HFO-1225yez; low molecular weight alcohols; low global warming potential olefins; chlorofluorocarbons; ketones; aldehydes; organic acids; and alkanes.
2. The mixture of Embodiment 1, wherein the additional compound comprises one or more of the compounds selected from the group consisting of cis-HFO-1234ze; HFO-1234yf; HFO 1225ye(Z); HFO 1225ye(E); HFO1225yc; HFO-1233zd; HFC-1233xf; (CF3)2CFCH=CHF (E & Z); (CF3)2CFCH=CF2; CF3CHFC=CHF (E & Z); and (C2F5)(CF3)C=CH2.
3. The mixture of Embodiment 1, wherein the additional compound comprises one or more of the compounds selected from the group consisting of HFC-245eb; HFC-245ca; HFC-227ea; HFC-236ea; HFC-236fa; HFC-134a; HFC-134; HFC-152a; HFC-32; HFC-125; HFC-143a; HFC-365mfc; HFC-161; and HFC-43-10mee.
4. The mixture of Embodiment 1, wherein the additional compound comprises one or more of the compounds selected from the group consisting of CHF2-O-CHF2;
   CHF2-O-CH2F; CH2F-O-CH2F; CH2F-O-CH3; cyclo-CF2-CH2-CF2-O; cyclo-CF2-CF2-CH2-O; CHF2-O-CF2-CHF2; CF3-CF2-O-CH2F; CHF2-O-CHF-CF3; CHF2-O-CF2-CHF2; CH2F-O-CF2-CHF2; CF3-O-CF2-CH3; CHF2-CHF-O-CHF2; CF3-O-CHF-CH2F; CF3-CHF-O-CH2F; CF3-O-CH2-CHF2; CHF2-O-CH2-CF3; CH2F-CF2-O-CH2F; CHF2-O-CF2-CH3; CHF2-CF2-O-CH3; CH2F-O-CHF-CH2F; CHF2-CHF-O-CH2F; CF3-O-CHF-CH3; CF3-CHF-O-CH3; CHF2-O-CH2-CHF2; CF3-O-CH2-CH2F; CF3-CH2-O-CH2F; and CF2H-CF2-CF2-O-CH3.
5. The mixture of Embodiment 1, wherein the additional compound comprises one or more of the compounds selected from the group consisting of propane; butane;
   isobutane; neopentane; isopentane; cyclopentane; n-hexane; isohexane; and heptane.
6. The mixture of Embodiment 1, for use as a blowing agent composition.
7. The blowing agent of Embodiment 6, useful for panel foams, comprising a mixture of HFO-1336mzzm and cyclopentane.
8. The blowing agent of Embodiment 6, useful for panel foams, comprising a mixture of HFO-1336mzzm and isopentane.
9. The blowing agent of Embodiment 6, useful for panel foams, comprising a mixture of HFO-1336mzzm and n-pentane.
10. The blowing agent of Embodiment 6, useful for spray foams, comprising a mixture of HFO-1336mzzm and HFC-245fa.
11. The blowing agent of Embodiment 6, useful for spray foams, comprising a mixture of HFO-1336mzzm and HFC-365mfc.
12. The blowing agent of Embodiment 6, useful for spray foams, comprising a mixture of HFO-1336mzzm and HFO-1233zd(E).
13. The mixture of Embodiment 1, for use as a polyol premix composition.
14. The mixture of Embodiment 1, for use as a foam composition.
15. The mixture of Embodiment 1, for use as a cleaning and contaminant removal composition.
16. The mixture of Embodiment 1, for use as a working fluid in an ORC power cycle.

## Claims

1. A foam made from a blowing agent composition comprising cis-1,1,1,4,4,4-hexafluoro-2-butene (Z-HFO-1336mzzm) and at least one additional compound selected from the group consisting of CO₂, formic acid, methyl formate and dimethoxymethane; wherein said composition comprises from 1 wt.% to 99 wt.% of said Z-HFO-1336mzzm.

2. The foam of claim 1, wherein the foam is a thermoset foam, preferably wherein the foam is a polyurethane foam, a polyisocyanurate foam or a phenolic foam.

3. The foam of claim 1, wherein the foam is a thermoplastic foam.

4. The foam of any preceding claim, wherein said blowing agent composition comprises Z-HFO-1336mzzm in an amount from 30 wt.% to 99 wt.%; from 50 wt.% to 99 wt.%; from 75 wt.% to 99 wt.%; from 85 wt.% to 99 wt.%; from 20 wt.% to 80 wt.%; from 90 wt.% to 99 wt.%; from 95 wt.% to 99 wt.%; from 1 wt.% to 20 wt.%; from 1 wt.% to 40 wt.%; from 1 wt.% to 50 wt.%; from 5 wt.% to 20 wt.%; from 5 wt.% to 40 wt.%; from 5 wt.% to 60 wt.%; from 10 wt.% to 80 wt.%; from 10 wt.% to 90 wt.%; from 20 wt.% to 90 wt.%.

5. The foam of any one of claims 1 to 3, wherein said blowing agent includes:
(i) formic acid in an amount of 1 to 99 wt.% or 10 to 90 wt.% or 10 to 80 wt.%; and/ or
(ii) methyl formate in an amount of 1 to 99 wt.% or 10 to 90 wt.% or 10 to 80 wt.%; and/ or
(iii) dimethoxymethane in an amount of 1 to 99 wt.% or 10 to 90 wt.% or 10 to 80 wt.%.

6. The foam of any preceding claim, wherein said blowing agent composition has an ozone depletion potential (ODP) of not greater than 0.5, preferably an ODP of not greater than 0.25, most preferably an ODP of not greater than 0.1; and/or a global warming potential (GWP) of not greater than 150, and even more preferably, a GWP of not greater than 50.

7. The foam of any preceding claim, wherein said blowing agent composition further comprises at least one co-blowing agent.

8. The foam of claim 7, wherein the at least one co-blowing agent is selected from the group consisting of water, CO₂, CFCs, HCCs, HCFCs, C1-C5 alcohols, C1-C4 aldehydes, C1-C4 ketones, C1-C4 ethers, C1-C4 HFCs, C4-C6 hydrocarbons and combinations of any of these.

9. The foam of claim 7, wherein the foam is a thermoplastic foam and the at least one co-blowing agent is selected from the group consisting of butane and isobutane.

10. The foam of claim 7, wherein the foam is a thermoset foam and the at least one co-blowing agent is selected from the group consisting of iso, normal and cyclopentane.

11. Use of a foam as defined in any preceding claim as an appliance foam selected from refrigerator foams, freezer foams, refrigerator/freezer foams and panel foams.

12. A blowing agent composition comprising cis-1,1,1,4,4,4-hexafluoro-2-butene (Z-HFO-1336mzzm) and at least one additional compound selected from the group consisting of CO₂, formic acid, methyl formate and dimethoxymethane; wherein said composition comprises from 1 wt.% to 99 wt.% of said Z-HFO-1336mzzm.

13. The blowing agent composition of claim 12, comprising Z-HFO-1336mzzm in an amount from 30 wt.% to 99 wt.%; from 50 wt.% to 99 wt.%; from 75 wt.% to 99 wt.%; from 85 wt.% to 99 wt.%; from 20 wt.% to 80 wt.%; from 90 wt.% to 99 wt.%; from 95 wt.% to 99 wt.%; from 1 wt.% to 20 wt.%; from 1 wt.% to 40 wt.%; from 1 wt.% to 50 wt.%; from 5 wt.% to 20 wt.%; from 5 wt.% to 40 wt.%; from 5 wt.% to 60 wt.%; from 10 wt.% to 80 wt.%; from 10 wt.% to 90 wt.%; from 20 wt.% to 90 wt.%.

14. The blowing agent composition of claim 12, wherein said blowing agent includes:
(i) formic acid in an amount of 1 to 99 wt.% or 10 to 90 wt.% or 10 to 80 wt.%; or
(ii) methyl formate in an amount of 1 to 99 wt.% or 10 to 90 wt.% or 10 to 80 wt.%; or
(iii) dimethoxymethane in an amount of 1 to 99 wt.% or 10 to 90 wt.% or 10 to 80 wt.%.

15. The blowing agent composition of any one of claims 12-14, wherein said blowing agent composition further comprises at least one co-blowing agent, preferably wherein the at least one co-blowing agent is selected from the group consisting of water, CO₂, CFCs, HCCs, HCFCs, C1-C5 alcohols, C1-C4 aldehydes, C1-C4 ketones, C1-C4 ethers, C1-C4 HFCs, C4-C6 hydrocarbons and combinations of any of these.

16. A method of forming a thermoset polyurethane or polyisocyanurate foam, the method comprising:
(a) providing an A component comprising an isocyanate;
(b) providing a B component comprising a polyol or polyol mixture, surfactant, catalysts, blowing agents, flame retardant, and other isocyanate reactive components; and
(c) bringing together the A and B side components either by hand mix for small preparations and, preferably, machine mix techniques to form blocks, slabs, laminates, pour-in-place panels and other items, spray applied foams and froths,
wherein the blowing agent composition is as defined in any one of claims 12 to 15.
